(19) Europäisches Patentamt
European Patent Office
Office européen des brevets

(11) **EP 3 469 986 B1**

(12) **FASCICULE DE BREVET EUROPEEN**

(45) Date de publication et mention
de la délivrance du brevet:
**03.11.2021 Bulletin 2021/44**

(21) Numéro de dépôt: **18199815.4**

(22) Date de dépôt: **11.10.2018**

(51) Int Cl.:
*A61B 5/06* (2006.01)        *A61B 5/107* (2006.01)
*A61B 5/00* (2006.01)        *G01C 25/00* (2006.01)
*G01R 33/00* (2006.01)       *G01V 3/08* (2006.01)

(54) **PROCEDE DE SYNCHRONISATION D'UN SYSTEME DE LOCALISATION MAGNETIQUE**

SYNCHRONISIERUNGSMETHODE FÜR EIN MAGNETISCHES ORTUNGSSYSTEM

METHOD OF SYNCHRONIZING A MAGNETIC LOCATION SYSTEM

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **13.10.2017 FR 1759637**

(43) Date de publication de la demande:
**17.04.2019 Bulletin 2019/16**

(73) Titulaire: **Commissariat à l'Énergie Atomique
et aux Énergies Alternatives
75015 Paris (FR)**

(72) Inventeurs:
• **JOSSELIN, Vincent
  38000 Grenoble (FR)**
• **BERTRAND, François
  38180 Seyssins (FR)**
• **ALOUI, Saifeddine
  38000 Grenoble (FR)**
• **PAULET, Jérôme
  26000 Valence (FR)**

(74) Mandataire: **INNOV-GROUP
310, avenue Berthelot
69372 Lyon Cedex 08 (FR)**

(56) Documents cités:
**US-A1- 2016 011 013    US-A1- 2017 131 424**

**Description**

**Domaine de l'invention**

Le domaine de l'invention concerne la localisation magnétique de dispositifs entre eux. Plus particulièrement, l'invention est relative à un procédé de synchronisation d'un système de localisation magnétique.

**Etat de la technique antérieure**

Un système de localisation magnétique peut se baser sur la modulation de champs magnétiques alternatifs, notamment en amplitude, pour déterminer une position relative et une orientation relative entre un dispositif comportant un émetteur de champs magnétiques alternatifs et un dispositif comportant un récepteur de champs magnétiques alternatifs.

Pour établir une localisation relative entre deux dispositifs, en particulier au moins l'un des dispositifs étant mobile par rapport à l'autre, il est connu d'équiper l'un des dispositifs de bobines émettrices et l'autre des dispositifs de bobines réceptrices. Les bobines émettrices permettent d'émettre des champs magnétiques alternatifs dont les modulations sont reçues par les bobines réceptrices. Ensuite, une analyse des champs magnétiques alternatifs reçus par les bobines réceptrices par rapport à des données relatives aux champs magnétiques alternatifs émis permet, via des démodulations et des filtrages adaptés, de déterminer la localisation relative entre les deux dispositifs. Une localisation relative peut correspondre à un calcul de la position et de l'orientation de l'un des dispositifs par rapport à l'autre des dispositifs. Le brevet US2017131424 décrit une application de localisation des objets en extérieur, ce qui lui permet de bénéficier d'un système GPS qui serait susceptible d'être utilisé pour réaliser une synchronisation de temps.

La demande de brevet US2005/0165297 décrit un exemple de fonctionnement d'un système de localisation magnétique.

La liaison entre les deux dispositifs peut être filaire. En particulier, avec une liaison filaire, on utilise un principe de détection synchrone par le dispositif réceptionnant les champs magnétiques alternatifs émis par le dispositif émettant les champs magnétiques alternatifs, notamment grâce à l'utilisation d'un signal pilote émis par le dispositif émettant les champs magnétiques alternatifs qui permet de caler temporellement la réception des champs magnétiques alternatifs à l'émission des champs magnétiques alternatifs : l'émission et la réception sont alors déclenchées simultanément. Ceci est notamment possible du fait de la présence d'un calculateur central comprenant un unique oscillateur pilotant de manière synchrone l'émission et la réception des champs magnétiques alternatifs.

Un inconvénient de la liaison filaire est la contrainte relative au lien physique entre les deux dispositifs, ou entre les deux dispositifs et un calculateur central, qui peut par exemple limiter les mouvements entre les deux dispositifs. Il existe donc un besoin de se libérer de la liaison filaire, par exemple, pour autoriser une meilleure ergonomie/souplesse d'utilisation du système de localisation magnétique.

Cependant, se libérer de la contrainte filaire n'est pas aussi simple. En effet, la suppression du lien filaire supprime de fait la référence de temps commune entre les deux dispositifs, chacun de ces dispositifs fonctionnant alors avec des oscillateurs indépendants. Il en résulte naturellement un inconvénient provoqué par une ambiguïté de phase dans le processus de démodulation des champs magnétiques reçus par le dispositif réceptionnant les champs magnétiques alternatifs, ambiguïté qu'il faut lever si l'on veut estimer de manière correcte l'orientation et la direction des champs magnétiques reçus, et donc estimer de manière correcte une localisation de l'un des dispositifs du système de localisation par rapport à l'autre des dispositifs du système de localisation. Plusieurs solutions existent pour pallier ce problème de synchronisation.

Une solution proposée par le brevet US8,121,812 est de mettre en place un multiplexage temporel des champs magnétiques émis, composé de trois fenêtres d'émission et une de synchronisation. Les fréquences des champs magnétiques émis sont par ailleurs contraintes : la durée des fenêtres d'émission doit correspondre à un multiple entier de la période de chaque champ magnétique émis. Cette solution présente l'inconvénient de limiter les fréquences utilisées pour mettre en œuvre la localisation magnétique et de ralentir globalement le système de localisation magnétique lié au multiplexage temporel des champs magnétiques. Par ailleurs, cette solution présente aussi l'inconvénient de limiter une levée d'ambiguïté de phase qu'à $\pi$ près, ceci ne permettant pas de détecter un décalage de phase, signe d'une présence d'un potentiel artefact gênant la localisation magnétique. Un artefact correspond à un perturbateur extérieur des champs magnétiques alternatifs.

La demande de brevet US2016/0011013 propose une solution pour lever l'ambiguïté de phase par synchronisation d'un émetteur et d'un récepteur de champs magnétiques. Pour cela, il est proposé dans cette demande de brevet US2016/0011013 une synchronisation permettant à une information de phase d'être initialement dérivée à partir d'informations connues. Ensuite, cette information de phase est suivie et mise à jour en supposant qu'il y a une corrélation entre les signaux du récepteur d'une mesure à l'autre. Cette solution présente différents inconvénients :

- elle nécessite une calibration initiale par apprentissage à partir de positions connues,
- si le signal est perdu au cours de la localisation magnétique, il faut réaliser à nouveau la phase de calibration,
- l'ambiguïté de la phase est limitée à $\pi$ près, ce qui

rend difficile l'application de méthodes de détection d'artefacts basées sur l'analyse fine des phases des champs magnétiques reçus.

Il existe donc un besoin de développer une solution, notamment plus robuste en cas de perte de signal, permettant une meilleure levée d'ambiguïté de phase, c'est-à-dire préférentiellement non limitée à π près.

**Objet de l'invention**

L'invention vise à remédier à tout ou partie des inconvénients précités. Notamment, l'invention a pour but de proposer une synchronisation permettant en particulier de maintenir identiques les phases et fréquences des modulations de champs magnétiques alternatifs émis avec des signaux de référence servant à la démodulation des champs magnétiques alternatifs reçus, en vue de mettre en œuvre une localisation magnétique.

A cet effet, un objet de l'invention est relatif à un procédé de synchronisation d'un système de localisation magnétique, le système de localisation magnétique comportant :

- un premier dispositif et un deuxième dispositif comportant chacun un oscillateur, un compteur de temps cadencé par ledit oscillateur, et un module de radiocommunication,
- un dispositif d'émission et de réception de champs magnétiques alternatifs configuré pour permettre une propagation de champs magnétiques alternatifs entre les premier et deuxième dispositifs, ledit dispositif d'émission et de réception de champs magnétiques alternatifs étant relié aux oscillateurs des premier et deuxième dispositifs,

le procédé de synchronisation comporte une étape de synchronisation configurée pour synchroniser les oscillateurs des premier et deuxième dispositifs par ajustement, en asservissant l'oscillateur du deuxième dispositif, du fonctionnement du compteur de temps du deuxième dispositif au fonctionnement du compteur de temps du premier dispositif.

Le procédé de synchronisation peut comporter une ou plusieurs des caractéristiques suivantes :

- l'étape de synchronisation comporte :

    - une étape de transmission de premiers horodatages, déterminés par le compteur de temps du premier dispositif, au deuxième dispositif par radiocommunication entre les modules de radiocommunication des premier et deuxième dispositifs,
    - une étape de détermination de deuxièmes horodatages par le compteur de temps du deuxième dispositif,
    - une étape d'asservissement de l'oscillateur du deuxième dispositif utilisant les premiers horodatages et les deuxièmes horodatages ;

- l'étape de synchronisation comporte des émissions de signaux de synchronisation par le module de radiocommunication du premier dispositif au module de radiocommunication du deuxième dispositif, chacun des premiers horodatages étant transmis par un des signaux de synchronisation, et chaque émission de signal de synchronisation par le module de radiocommunication du premier dispositif provoque :

    - une détermination, par le compteur de temps du premier dispositif, d'un des premiers horodatages à transmettre par un signal de synchronisation consécutif audit signal de synchronisation émis,
    - une détermination, lors d'une réception par le module de radiocommunication du deuxième dispositif dudit signal de synchronisation émis, d'un des deuxièmes horodatages par le compteur de temps du deuxième dispositif ;

- l'étape d'asservissement comporte des étapes de détermination d'une consigne d'asservissement de l'oscillateur du deuxième dispositif chacune suivie d'une étape d'application de ladite consigne d'asservissement, chaque consigne d'asservissement étant déterminée en utilisant un différentiel temporel prenant en compte un des premiers horodatages et un des deuxièmes horodatages ;

- l'étape d'asservissement comporte des étapes de détermination d'une consigne d'asservissement de l'oscillateur du deuxième dispositif chacune suivie d'une étape d'application de ladite consigne d'asservissement, chaque consigne d'asservissement étant déterminée en utilisant un différentiel temporel prenant en compte un des premiers horodatages et un des deuxièmes horodatages, correspondant respectivement à un horodatage de l'émission et à un horodatage de la réception d'un même signal de synchronisation ;

- chaque étape de détermination d'une consigne d'asservissement de l'oscillateur du deuxième dispositif est déclenchée par la réception d'un des signaux de synchronisation, et les signaux de synchronisation sont émis à intervalles réguliers de sorte à permettre l'application de la consigne d'asservissement déterminée entre la réception du signal de synchronisation ayant déclenché sa détermination et la réception d'un signal de synchronisation consécutif ;

- chaque signal de synchronisation reçu par le module de radiocommunication du deuxième dispositif, et pour lequel le deuxième dispositif dispose d'un des deuxièmes horodatages déterminé par le compteur de temps du deuxième dispositif lors de la réception d'un signal de synchronisation précédent audit signal de synchronisation reçu, provoque la mise en œuvre de l'une des étapes de détermination d'une consigne d'asservissement pour laquelle le différentiel temporel est obtenu en prenant en compte :

- un des premiers horodatages extrait dudit signal de synchronisation reçu, et
- ledit deuxième horodatage déterminé par le compteur de temps du deuxième dispositif lors de la réception dudit signal de synchronisation précédent audit signal de synchronisation reçu ;

• l'étape d'asservissement de l'oscillateur du deuxième dispositif comporte une utilisation d'un filtre de boucle ;
• le filtre de boucle implémente une loi de commande proportionnelle-intégrale ;
• chaque consigne d'asservissement est une tension de contrôle de l'oscillateur du deuxième dispositif.

L'invention est aussi relative à un procédé de localisation magnétique comportant une étape de mise en œuvre du procédé de synchronisation tel que décrit, et une étape de localisation relative entre les premier et deuxième dispositifs en prenant en compte des données issues du dispositif d'émission et de réception de champs magnétiques alternatifs.

Le procédé de localisation magnétique peut comporter une ou plusieurs des caractéristiques suivantes :

• le procédé de localisation magnétique comporte, après ajustement du fonctionnement du compteur de temps du deuxième dispositif au fonctionnement du compteur de temps du premier dispositif, une étape de comparaison d'une consigne temporelle avec des données issues des compteurs de temps des premier et deuxième dispositifs, et le procédé de localisation magnétique comporte une étape de démarrage du dispositif d'émission et de réception de champs magnétiques alternatifs déclenchée en fonction du résultat de l'étape de comparaison ;
• le dispositif d'émission et de réception de champs magnétiques alternatifs comporte un émetteur de champs magnétiques alternatifs et un récepteur de champs magnétiques alternatifs, et le procédé de localisation magnétique comporte, après synchronisation des oscillateurs des premier et deuxième dispositifs :

  - une étape d'activation de l'émetteur de champs magnétiques alternatifs, cadencé par l'un des oscillateurs des premier et deuxième dispositifs, d'où il résulte l'émission de champs magnétiques alternatifs, et
  - une étape d'activation du récepteur de champs magnétiques alternatifs, cadencé par l'autre des oscillateurs des premier et deuxième dispositifs, d'où il résulte la réception des champs magnétiques alternatifs émis,

  les étapes d'activation des récepteur et émetteur de champs magnétiques alternatifs étant déclenchées simultanément en utilisant une consigne temporelle comparée, d'une part, à une donnée de sortie du compteur de temps du premier dispositif, et, d'autre part, à une donnée de sortie du compteur de temps du deuxième dispositif ;
• l'étape de synchronisation est déclenchée avant d'activer les émetteur et récepteur, et est maintenue active au cours du fonctionnement des émetteur et récepteur.

L'invention est aussi relative à un système de localisation magnétique comportant :

- un premier dispositif et un deuxième dispositif comportant chacun un oscillateur, un compteur de temps cadencé par ledit oscillateur, et un module de radiocommunication,
- un dispositif d'émission et de réception de champs magnétiques alternatifs configuré pour permettre une propagation de champs magnétiques alternatifs entre les premier et deuxième dispositifs, ledit dispositif d'émission et de réception de champs magnétiques alternatifs étant relié aux oscillateurs des premier et deuxième dispositifs,

le système de localisation est configuré de sorte à permettre une synchronisation des oscillateurs des premier et deuxième dispositifs par ajustement, en asservissant l'oscillateur du deuxième dispositif, du fonctionnement du compteur de temps du deuxième dispositif au fonctionnement du compteur de temps du premier dispositif.

**Description sommaire des dessins**

D'autres avantages et caractéristiques ressortiront clairement de la description qui va suivre de modes particuliers de réalisation de l'invention donnés à titre d'exemples non limitatifs et représentés sur les dessins annexés, dans lesquels :

- La figure 1 représente un mode de réalisation d'un premier dispositif d'un système de localisation magnétique, de préférence destiné à émettre des champs magnétiques alternatifs en vue de permettre une localisation relative entre le premier dispositif et un deuxième dispositif ;
- La figure 2 représente un mode de réalisation du deuxième dispositif du système de localisation magnétique, de préférence destiné à recevoir les champs magnétiques alternatifs qui seront émis par le premier dispositif en vue de permettre la localisation relative entre le premier dispositif et le deuxième dispositif ;
- La figure 3 illustre un mode de réalisation d'un procédé de synchronisation du système de localisation magnétique ;

- La figure 4 illustre, selon un mode de réalisation, le fonctionnement des premier et deuxième dispositifs lors de la transmission de signaux de synchronisation entre les premier et deuxième dispositifs ;
- La figure 5 illustre un mode de réalisation d'un procédé de localisation magnétique.

Dans ces figures, les mêmes références sont utilisées pour désigner les mêmes éléments.

## Description de modes particuliers de réalisation

Un objet de l'invention est relatif à un procédé de synchronisation d'un système de localisation magnétique permettant de synchroniser deux oscillateurs distincts qui seront utilisés pour cadencer un dispositif d'émission et de réception de champs magnétiques alternatifs, ces champs magnétiques alternatifs étant destinés à être utilisés pour déterminer une localisation relative entre des premier et deuxième dispositifs du système de localisation magnétique qui seront décrits plus en détails ci-après.

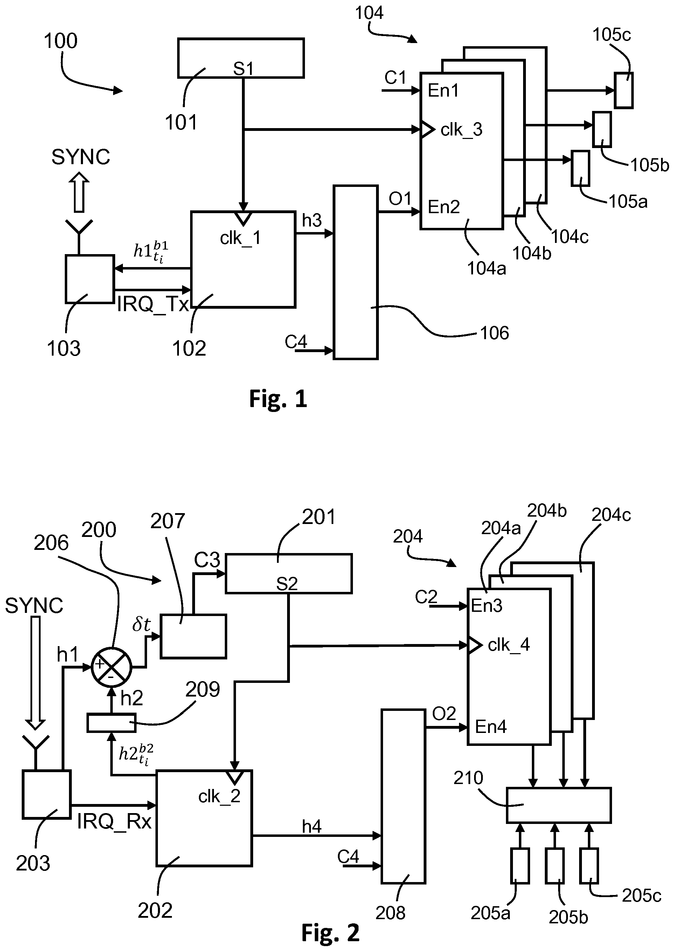

Notamment, des parties du système de localisation magnétique sont illustrées en figures 1 et 2. Le système de localisation magnétique comporte un premier dispositif 100 (figure 1) et un deuxième dispositif 200 (figure 2). Le système de localisation magnétique est configuré pour établir au moins une localisation relative entre les premier et deuxième dispositifs 100, 200, notamment à partir de champs magnétiques alternatifs s'étant propagés entre les premier et deuxième dispositifs 100, 200. Autrement dit, des données issues des, c'est-à-dire relatives aux, champs magnétiques alternatifs, notamment émis et reçus, peuvent être utilisées/prises en compte pour permettre de déterminer une localisation relative entre les premier et deuxième dispositifs 100, 200.

Par « localisation relative entre les premier et deuxième dispositifs 100, 200 », on entend la localisation de l'un des premier et deuxième dispositifs 100, 200 par rapport à l'autre des premier et deuxième dispositifs 100, 200.

La localisation peut correspondre dans le domaine de la présente invention à un positionnement et/ou à une orientation particulière de l'un des premier et deuxième dispositifs 100, 200 par rapport à l'autre des premier et deuxième dispositifs 100, 200.

Ainsi, en établissant une pluralité de localisations relatives entre les premier et deuxième dispositifs 100, 200 à des instants différents, il est possible de suivre le déplacement de l'un des premier et deuxième dispositifs 100, 200 par rapport à l'autre des premier et deuxième dispositifs 100, 200.

Sur la figure 1, le premier dispositif 100 comporte un oscillateur 101, un compteur de temps 102 cadencé par l'oscillateur 101 du premier dispositif 100, et un module de radiocommunication 103.

Sur la figure 2, le deuxième dispositif 200 comporte un oscillateur 201, un compteur de temps 202 cadencé par l'oscillateur 201 du deuxième dispositif 200, et un module de radiocommunication 203.

Par « oscillateur » 101, 201, on entend un composant électronique dont la fonction est de produire un signal périodique destiné à cadencer d'autres composants électroniques, comme par exemple un compteur de temps, un synthétiseur de fréquence, etc.

Par « compteur de temps » 102, 202, on entend un composant électronique dont la fonction est de fournir un horodatage. Le cadencement d'un compteur de temps est mis en œuvre par l'oscillateur auquel il est relié. Par exemple, le compteur de temps 102 du premier dispositif 100 comporte une entrée d'horloge clk_1 reliée à une sortie de cadencement S1 de l'oscillateur 101 du premier dispositif 100 (figure 1). Par exemple, le compteur de temps 202 du deuxième dispositif 200 comporte une entrée d'horloge clk_2 reliée à une sortie de cadencement S2 de l'oscillateur 201 du deuxième dispositif 200 (figure 2). Par exemple, les entrées d'horloge permettent de fournir des impulsions à dénombrer dans le cadre des compteurs de temps.

Par « horodatage », appelé « timestamp » en langue anglaise, on entend une donnée qui permet d'associer une information temporelle à un événement. De manière générale, un horodatage peut représenter une quantité de temps écoulée depuis un instant de référence, ou encore une date et une heure.

Bien entendu, les modules de radiocommunication 103, 203 sont configurés de sorte à entrer en communication sans fil pour échanger des données. Un module de radiocommunication au sens de la présente description est donc un module émetteur-récepteur d'ondes radioélectriques.

De préférence, le système de localisation magnétique est tel que l'un des premier et deuxième dispositifs 100, 200 est apte à se déplacer par rapport à l'autre des premier et deuxième dispositifs 100, 200. Par ailleurs, il n'existe pas de lien de communication filaire entre les premier et deuxième dispositifs 100, 200.

Par ailleurs, le système de localisation magnétique comporte aussi le dispositif d'émission et de réception 104, 204 de champs magnétiques alternatifs relié aux oscillateurs 101, 201 des premier et deuxième dispositifs 100, 200. Ce dispositif d'émission et de réception 104, 204 est configuré pour permettre une propagation de champs magnétiques alternatifs entre le premier dispositif 100 et le deuxième dispositif 200, notamment en vue de mettre en œuvre au moins une étape de localisation magnétique relative entre le premier dispositif 100 et le deuxième dispositif 200 en prenant en compte des données issues du dispositif d'émission et de réception 104, 204 comme il le sera décrit ci-après. Ce dispositif d'émission et de réception 104, 204 de champs magnétiques alternatifs est notamment formé en deux parties dont l'une est intégrée au premier dispositif 100 et l'autre au deuxième dispositif 200. Ces parties peuvent être respectivement formées par un émetteur 104 de champs magnétiques alternatifs et un récepteur 204 de champs magnétiques alternatifs. L'émetteur 104 de champs magnétiques alternatifs peut être cadencé par l'un des oscillateurs (celui du premier dispositif 100 dans l'exemple illustré en figure 1) et le récepteur 204 de champs magnétiques alternatifs peut alors cadencé par l'autre des oscillateurs (celui du deuxième dispositif 200 dans l'exemple illustré en figure 2). On comprend donc qu'il existe un problème de synchronisation à résoudre si l'on veut améliorer le fonctionnement du système de localisation magnétique puisque l'émetteur 104 et le récepteur 204 seront cadencés par des oscillateurs distincts.

L'émetteur 104 de champs magnétiques alternatifs peut comporter des synthétiseurs de fréquence 104a, 104b, 104c, aussi appelés synthétiseurs numériques directs et connus sous le sigle DDS correspondant en langue anglaise à « Direct Digital Synthesizer ». Chaque synthétiseur de fréquence 104a, 104b, 104c de l'émetteur 104 de champs magnétiques alternatifs peut comporter une entrée d'horloge clk_3 reliée à la sortie de cadencement S1 de l'oscillateur 101 du premier dispositif 100, une entrée En1 de consigne C1 lui précisant sur quelle fréquence il doit fonctionner, et une entrée En2 destinée à recevoir un ordre de démarrage O1. Pour des raisons de clarté de la figure 1, seules les entrées En1 de consigne C1, En2 d'ordre de démarrage O1 et d'horloge clk_3 du synthétiseur de fréquence 104a sont représentées, mais le même principe s'applique aux autres synthétiseurs de fréquence 104b, 104c. Chaque synthétiseur de fréquence 104a, 104b, 104c pilote une bobine 105a, 105b, 105c de l'émetteur 104 destinée à générer un champ magnétique alternatif correspondant selon un pilotage adéquat du synthétiseur de fréquence associé. Selon une autre formulation, chaque bobine 105a, 105b, 105c est excitée avec une fréquence qui lui est propre par un synthétiseur de fréquence 104a, 104b, 104c associé à ladite bobine 105a, 105b, 105c.

Le récepteur 204 de champs magnétiques alternatifs peut comporter des synthétiseurs de fréquence 204a, 204b, 204c. Chaque synthétiseur de fréquence 204a, 204b, 204c du récepteur 204 de champs magnétiques alternatifs peut comporter une entrée d'horloge clk_4 reliée à la sortie de cadencement S2 de l'oscillateur 201 du deuxième dispositif 200, une entrée En3 de consigne C2 lui précisant sur quelle fréquence il doit fonctionner, et une entrée En4 destinée à recevoir un ordre de démarrage 02. Chaque synthétiseur de fréquence 204a, 204b, 204c permet de fournir une fréquence de modulation de référence, par exemple une phase et une quadrature à une unité de démodulation 210 des champs magnétiques alternatifs reçus. Cette unité de démodulation 210 est reliée à des bobines 205a, 205b, 205c (dites « bobines réceptrices ») du récepteur 204 de champs magnétiques alternatifs destinées à capter/recevoir les champs magnétiques alternatifs correspondants émis par les bobines de l'émetteur 104 de champs magnétiques alternatifs. La phase et la quadrature permettent de remonter à deux informations à savoir l'amplitude et la phase du signal démodulé sur l'ensemble des bobines réceptrices 205a, 205b, 205c. Pour chaque bobine 205a, 205b, 205c du récepteur 204, il est fourni une phase et une quadrature pour chaque fréquence. Ainsi, par exemple, pour un récepteur tri-axe recevant des signaux émis par trois bobines 105a, 105b, 105c, il y a neuf couples de phase et de quadrature à utiliser par l'unité de démodulation 210. Ainsi, l'unité de démodulation 210 permet de réaliser des détections synchrones en phases et en quadratures pour retrouver, du côté du récepteur 204 de champs magnétiques alternatifs, les amplitudes et les phases des champs magnétiques reçus à partir d'informations fournies par les synthétiseurs de fréquence 204a, 204b, 204c du récepteur 204 de champs magnétiques alternatifs. Pour des raisons de clarté de la figure 2, seules les entrées En3 de consigne C2, En4 d'ordre de démarrage 02 et d'horloge clk_4 du synthétiseur de fréquence 104a sont représentées en figure 2, mais le même principe s'applique aux autres synthétiseurs de fréquence 204b, 204c.

Les consignes C1 sont différentes pour chaque synthétiseur de fréquence 104a, 104b, 104c de l'émetteur 104 de champs magnétiques alternatifs dans le but de différencier les fréquences des champs magnétiques émis par les différentes bobines 105a, 105b, 105c de l'émetteur 104 de champs magnétiques alternatifs. On comprend alors que chaque consigne C1 est différente des autres consignes C1. Par ailleurs, chaque consigne C2 est identique à une des consignes C1, les consignes C1 peuvent être choisies par le premier dispositif 100, et transmises au deuxième dispositif 200 par radiocommunication entre les modules de radiocommunication 103, 203 des premier et deuxième dispositifs 100, 200. On comprend alors que chaque consigne C2 est différente des autres consignes C2.

Sur les figures 1 et 2, il est représenté un mode de réalisation pour lequel le premier dispositif 100 comporte l'émetteur 104 de champs magnétiques alternatifs, et pour lequel le deuxième dispositif 200 comporte le récepteur 204 de champs magnétiques alternatifs. Bien que l'inverse soit possible, ce mode de réalisation est préféré car le récepteur 204 de champs magnétiques alternatifs (ou plus généralement le deuxième dispositif 200) est généralement mobile impliquant que le deuxième dispositif 200 soit plus compact : on y intègre donc par exemple une source d'énergie et la réception des champs magnétiques alternatifs qui consomme moins d'énergie que leur émission.

L'utilisation de données relatives aux champs magnétiques alternatifs reçus et émis pour permettre de générer au moins une localisation magnétique relative entre les premier et deuxième dispositifs 100, 200 ne sera pas décrite en détails car connue en soi par l'homme du métier, par exemple une telle utilisation peut être dérivée de la demande de brevet US2005/0165297, ou encore de l'article de RAAB et al. intitulé « Magnetic Position and Orientation Tracking System » publié dans IEEE Transactions on aerospace and electronic systems , Vol. AES-15, No. 5 en septembre 1979 pages 709 à 718.

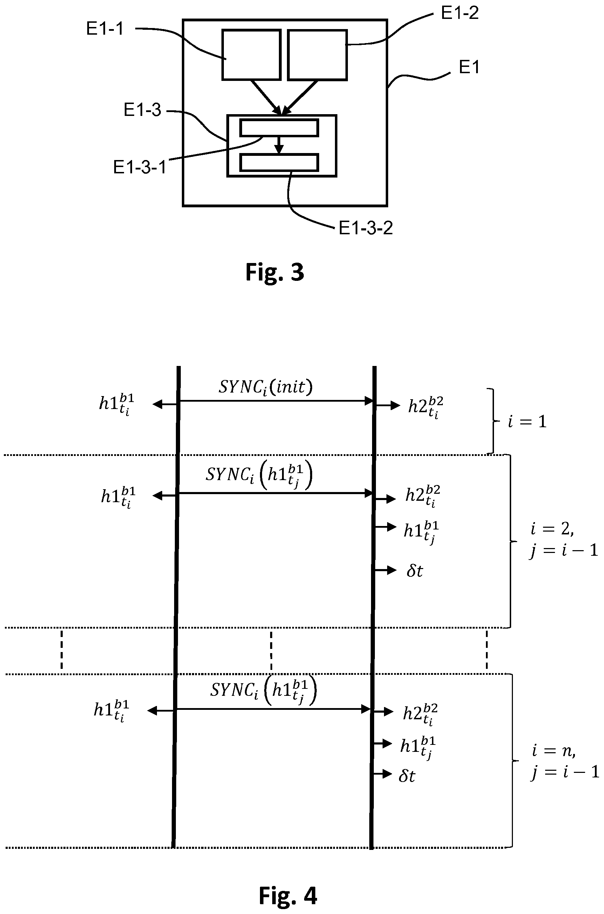

Comme le montre la figure 3, le procédé de synchronisation du système de localisation magnétique comporte une synchronisation des, c'est-à-dire une étape de synchronisation E1 configurée pour synchroniser les, oscillateurs 101, 201 des premier et deuxième dispositifs 100, 200 par ajustement du fonctionnement du compteur de temps 202 du deuxième dispositif 200 au fonctionnement du compteur de temps 102 du premier dispositif 100. Notamment, cet ajustement est réalisé en asservissant l'oscillateur 201 du deuxième dispositif 200. Par asservissement de l'oscillateur 201 du deuxième dispositif 200, on entend que son fonctionnement va être modifié volontairement du fait de l'ajustement recherché entre les compteurs de temps, alors que l'oscillateur 101 du premier dispositif 100 restera préférentiellement à une même fréquence de fonctionnement : la fréquence de l'oscillateur 201 peut être modifiée alors que celle de l'oscillateur 101 ne l'est préférentiellement pas. L'ajustement du fonctionnement d'un compteur de temps à un autre permet à ces derniers de tendre à donner, pour un même instant, des horodatages similaires/identiques selon un décalage résiduel adéquat, typiquement inférieur à la microseconde. En particulier, la stabilité de la synchronisation peut être telle que la différence d'horodatage entre les compteurs de temps 102, 202 des premier et deuxième dispositifs 100, 200 peut être comprise entre -100ns et 100ns (ceci étant fonction de la résolution des compteurs de temps ou du bruit associé au fonctionnement de puces de radiocommunication utilisées dans les modules de radiocommunication pour traiter les radiocommunications participant à la synchronisation telle qu'elle sera décrite ci-après) lorsque la synchronisation est effective. Par stabilité de la synchronisation, on entend qu'elle peut être reproductible à chaque démarrage du système de localisation magnétique, et donc à chaque synchronisation des oscillateurs consécutive audit démarrage. Autrement dit, l'ajustement du fonctionnement du compteur de temps 202 du deuxième dispositif 200 au fonctionnement du compteur de temps 102 du premier dispositif 100 permet de faire fonctionner les compteurs de temps selon une même base temporelle (par exemple en date et en heure) tout en synchronisant les oscillateurs, ceci a pour corollaire que l'étape de synchronisation est telle qu'elle permet de faire fonctionner les compteurs de temps selon une même base temporelle. Par la suite, la base temporelle de l'oscillateur 101 du premier dispositif 100 est notée b1, et la base temporelle de l'oscillateur 201 du deuxième dispositif 200 est notée b2.

La synchronisation est ici matérielle car elle agit directement sur le fonctionnement d'un des oscillateurs. En choisissant des composants adaptés, la précision de la synchronisation est satisfaisante dans l'application recherchée de la localisation magnétique.

Il résulte de cette étape de synchronisation E1 qu'il est ensuite possible de mettre en œuvre une localisation magnétique de manière précise car le système de localisation magnétique dispose de premier et deuxième dispositifs 100, 200 (notamment dont l'un est à localiser par rapport à l'autre) dont les oscillateurs 101, 201, utilisés par le dispositif d'émission et de réception 104, 204 de champs magnétiques alternatifs, sont synchronisés, et dont les compteurs de temps 102, 202 sont ajustés l'un à l'autre. En ce sens, il est possible de maîtriser l'instant de démarrage du dispositif d'émission et de réception 104, 204 de champs magnétiques alternatifs tant côté émetteur 104 que côté récepteur 204, ainsi que la fréquence de fonctionnement du dispositif d'émission et de réception 104, 204 de champs magnétiques alternatifs. Autrement dit, l'ajustement décrit présente l'avantage, d'une part, d'obtenir un système de datation commun à des premier et deuxième dispositifs 100, 200 distincts comportant chacun son compteur de temps cadencé par un oscillateur associé, et, d'autre part, de synchroniser les oscillateurs (côté émetteur et côté récepteur) à des fréquences de fonctionnement d'où il résulte la possibilité de mettre en œuvre une localisation magnétique en maitrisant la phase d'origine de signaux alternatifs utilisés pour émettre les champs magnétiques alternatifs ainsi que la phase d'origine de signaux alternatifs générés par les bobines réceptrices : la limitation à $\pi$ près est levée. Côté émetteur 104, les signaux alternatifs sont utilisés pour exciter les bobines 105a, 105b, 105c, dites bobines émettrices, d'où il résulte l'émission des champs magnétiques alternatifs. Côté récepteur 204, les bobines réceptrices 205a, 205b, 205c sont excitées par des champs d'excitation issus des champs magnétiques alternatifs émis d'où il résulte la génération, pour chaque bobine réceptrice, d'un signal alternatif en vue de réaliser la localisation magnétique. Ainsi, la synchronisation permet en particulier de maintenir identiques les phases et fréquences des modulations de champs magnétiques alternatifs émis avec des signaux de référence servant à la démodulation des champs magnétiques alternatifs reçus. Les signaux de référence sont construits côté récepteur 204 en utilisant l'oscillateur 201 du deuxième dispositif 200 synchronisé à celui du premier dispositif 100 : on dispose alors d'une même phase d'origine et d'une même fréquence pour chacun des premier et deuxième dispositifs 100, 200. Les signaux de référence permettent de décoder les signaux alternatifs générés par les bobines réceptrices 205a, 205b, 205c pour ensuite mettre en œuvre, à partir des données décodées, la localisation magnétique. En ce sens, il est proposé une solution permettant que l'émission et la réception des champs magnétiques alternatifs se fassent de manière synchrone : il est donc obtenu la robustesse d'une solution filaire en pilotant l'émission et la réception des champs magnétiques alternatifs, tout en bénéficiant de l'ergonomie/de la souplesse d'utilisation d'une solution sans-fil.

De préférence, il est utilisé les modules de radiocommunication 103, 203 pour transmettre des données permettant de participer à la synchronisation puisque les premier et deuxième dispositifs 100, 200 ne peuvent pas communiquer par liaison filaire entre eux. En ce sens, l'étape de synchronisation E1 peut comporter une étape de transmission E1-1 de premiers horodatages $h1_{t_i}^{b1}$, déterminés par le compteur de temps 102 du premier dispositif 100, au deuxième dispositif 200 par radiocommunication entre les modules de radiocommunication 103, 203 des premier et deuxième dispositifs 100, 200. Les premiers horodatages sont donc transmis au module de radiocommunication 203 du deuxième dispositif 200 par le module de radiocommunication 103 du premier dispositif 100. Selon la notation $h1_{t_i}^{b1}$, $i$ représente un entier positif supérieur ou égal à 1 et permettant d'identifier le premier horodatage correspondant, $b1$ fait référence à la base temporelle du compteur de temps 102 du premier dispositif 100. Il en résulte que le deuxième dispositif 200 reçoit, notamment successivement et notamment à intervalles réguliers, les premiers horodatages $h1_{t_i}^{b1}$ qu'il pourra utiliser pour asservir son oscillateur 201. L'étape de synchronisation E1 comporte aussi une étape de détermination E1-2 de deuxièmes horodatages $h2_{t_i}^{b2}$ par le compteur de temps 202 du deuxième dispositif 200. Selon la notation $h2_{t_i}^{b2}$, $i$ représente un entier positif supérieur ou égal à 1 et permettant d'identifier le deuxième horodatage correspondant, b2 fait référence à la base temporelle du compteur de temps 202 du deuxième dispositif 200. Par ailleurs, l'étape de synchronisation E1 comporte en outre une étape d'asservissement E1-3 de l'oscillateur 201 du deuxième dispositif 200 utilisant, c'est-à-dire prenant en compte, les premiers horodatages $h1_{t_i}^{b1}$ et les deuxièmes horodatages $h2_{t_i}^{b2}$. Cette étape d'asservissement E1-3 est mise en œuvre par le deuxième dispositif 200. En fait, les différents premiers horodatages pourront être utilisés successivement en association avec des deuxièmes horodatages correspondants pour modifier le fonctionnement de l'oscillateur 201 du deuxième dispositif 200, par exemple en l'accélérant ou en le ralentissant, de sorte à tendre à l'obtention d'un système de datation commun et à la synchronisation des oscillateurs 101, 201 des premier et deuxième dispositifs 100, 200. Une telle utilisation d'horodatages est avantageuse car elle permet de faciliter la synchronisation des oscillateurs puisque les compteurs de temps sont eux même cadencés par lesdits oscillateurs.

En ce sens, l'étape d'asservissement E1-3 peut être telle que le fonctionnement de l'oscillateur 201 du deuxième dispositif 200 va être modifié pour agir sur le cadencement du compteur de temps 202 du deuxième dispositif 200 afin de caler le fonctionnement dudit compteur de temps 202 du deuxième dispositif 200 sur celui du premier dispositif 100 d'où il résultera par ailleurs la synchronisation des oscillateurs 101, 201 des premier et deuxième dispositifs 100, 200. Selon un exemple préféré, l'étape d'asservissement E1-3 peut comporter au moins une ou des étape(s) de détermination E1-3-1 d'une consigne d'asservissement C3 (figure 2) de l'oscillateur 201 du deuxième dispositif 200 chacune suivie d'une étape d'application E1-3-2 de ladite consigne d'asservissement C3 à l'oscillateur 201 du deuxième dispositif 200. Ladite, et le cas échéant chaque, consigne d'asservissement C3 est déterminée en utilisant un (c'est-à-dire à partir d'un) différentiel temporel $\delta t$ (figure 2) prenant en compte un des premiers horodatages (représenté par la référence h1 à la figure 2) et un des deuxièmes horodatages (représenté par la référence h2 à la figure 2). Pour générer le différentiel temporel $\delta t$, le deuxième dispositif 200 peut comporter un générateur de différentiel 206 (figure 2) prenant en entrée deux horodatages (ici représentés h1 et h2) et générant en sortie le différentiel temporel noté $\delta t$. L'utilisation d'un différentiel temporel est avantageuse dans le sens où il s'agit d'une donnée simple à exploiter dans le cadre de l'ajustement recherché notamment mis en œuvre par asservissement d'un des oscillateurs, de préférence sur la base d'une loi de commande PI (proportionnelle-intégrale), à accélérer ou à ralentir. Ainsi, l'étape de synchronisation peut comporter une étape de détermination du différentiel temporel $\delta t$.

Dans la présente description, de manière générale, la - ou chaque - consigne d'asservissement est préférentiellement déterminée de sorte à agir sur l'écart d'horodatage entre les premier et deuxième compteurs de temps 102, 202. Notamment, la - ou chaque - consigne d'asservissement est préférentiellement déterminée de sorte à diminuer l'écart d'horodatage entre les premier et deuxième compteurs de temps 102, 202, ou de sorte à maintenir la valeur absolue de cet écart en dessous d'un seuil prédéterminé. Le seuil prédéterminé est alors choisi en fonction de l'application pour laquelle il est considéré que les oscillateurs 101, 201 des premier et deuxième dispositifs 100, 200 sont synchronisés.

On comprend que l'ajustement du fonctionnement du compteur de temps 202 du deuxième dispositif 200 au fonctionnement du compteur de temps 102 du premier dispositif 100 (et donc à terme la synchronisation des oscillateurs 101, 201) peut être mise en œuvre en utilisant une comparaison de premier et deuxième horodatages $h1_{t_i}^{b1}$, $h2_{t_i}^{b2}$ liés entre eux. L'idée est donc ici de déterminer un des premiers horodatages $h1_{t_i}^{b1}$, selon la base temporelle du compteur de temps 102 du premier dispositif 100, et un des deuxième horodatages $h2_{t_i}^{b2}$, selon la base temporelle du compteur de temps 202 du deuxième dispositif 200, à des instants proches ou similaires pour pouvoir les comparer en vue d'asservir le fonctionnement de l'oscillateur 201 du deuxième dispositif 200 par l'ajustement évoqué précédemment. Une solution efficace pour mettre en œuvre cette idée propose d'utiliser le fait que le délai de transmission d'un signal depuis le module de radiocommunication 103 du premier dispositif 100 vers le module de radiocommunication 203 du deuxième dispositif 200, ainsi que la transition entre un niveau bas (0) et un niveau haut (1) de ce signal sont suffisamment déterministes pour permettre de comparer un horodatage généré lors de l'émission d'un signal du côté du premier dispositif 100 avec un horodatage généré lors de la réception de ce même signal du côté du deuxième dispositif 200. Ceci présente l'avantage d'obtenir des valeurs cohérentes à fournir comme entrées dans le cadre d'un asservissement de l'oscillateur du deuxième dispositif 200.

On comprend alors qu'il est préférentiellement possible d'associer un instant d'émission (figure 1) d'un signal de synchronisation SYNC, par le module de radiocommunication 103 du premier dispositif 100, à un des premiers horodatages $h1_{t_i}^{b1}$, et un instant de réception (en figure 2) de ce signal de synchronisation SYNC, par le module de radiocommunication 203 du deuxième dispositif 200, à un des deuxièmes horodatages $h2_{t_i}^{b2}$ qui pourra être comparé par la suite audit premier horodatage $h1_{t_i}^{b1}$ pour établir le différentiel temporel.

Pour être le plus précis possible, il est préféré que, pour chaque premier horodatage $h1_{t_i}^{b1}$, la détermination dudit premier horodatage $h1_{t_i}^{b1}$ se fasse lors de l'émission d'un signal de synchronisation SYNC correspondant, et que pour chaque deuxième horodatage $h2_{t_i}^{b2}$, la détermination dudit deuxième horodatage se fasse lors de la réception d'un signal de synchronisation correspondant. En ce sens, au moment de la réception d'un signal de synchronisation SYNC permettant de déterminer un des deuxièmes horodatages $h2_{t_i}^{b2}$, le deuxième dispositif 200 ne dispose pas du premier horodatage $h1_{t_i}^{b1}$ associé à comparer avec ledit un des deuxièmes horodatages $h2_{t_i}^{b2}$ pour déterminer le différentiel temporel $\delta t$ évoqué précédemment. Il existe donc un besoin de développer un mécanisme permettant de prendre en compte deux horodatages correspondant respectivement à l'émission d'un signal par le premier dispositif 100 et à la réception de ce même signal par le deuxième dispositif 200 pour déterminer un différentiel temporel correspondant. Les deux horodatages que l'on cherche à comparer sont dits « liés » ou « correspondants ».

La solution proposée au besoin de développer le mécanisme est la suivante : l'étape de synchronisation E1 peut comporter des émissions de signaux de synchronisation SYNC par le module de radiocommunication 103 du premier dispositif 100 au module de radiocommunication 203 du deuxième dispositif 200. Ces émissions de signaux de synchronisation SYNC peuvent se faire à intervalles réguliers ce qui permet d'améliorer la convergence du fonctionnement du compteur de temps 202 du deuxième dispositif 200 vers celui du premier dispositif 100. Ici chacun des premiers horodatages $h1_{t_i}^{b1}$ est transmis par un des signaux de synchronisation SYNC, le premier horodatage à transmettre peut alors être encapsulé dans un paquet de données que le signal de synchronisation propage du module de radiocommunication 103 du premier dispositif 100 au module de radiocommunication 203 du deuxième dispositif 200. Par ailleurs, chaque émission de signal de synchronisation SYNC en figure 1 par le module de radiocommunication 103 du premier dispositif 100 provoque :

- une détermination, par le compteur de temps 102 du premier dispositif 100, d'un des premiers horodatages $h1_{t_i}^{b1}$ à transmettre par un signal de synchronisation consécutif audit signal de synchronisation émis SYNC, et
- une détermination, lors d'une réception par le module de radiocommunication 203 du deuxième dispositif 200 dudit signal de synchronisation émis SYNC, d'un des deuxièmes horodatages $h2_{t_i}^{b2}$ par le compteur de temps 202 du deuxième dispositif 200.

Ainsi, il est déterminé de manière cohérente des premiers et deuxièmes horodatages $h1_{t_i}^{b1}$, $h2_{t_i}^{b2}$ tout en transmettant les premiers horodatages par des signaux de synchronisation émis successivement au deuxième dispositif 200 afin que celui-ci puisse mettre en œuvre l'asservissement de son oscillateur 201. Ainsi, lorsqu'un des deuxièmes horodatages $h2_{t_i}^{b2}$ est déterminé, il pourra être comparé au premier horodatage $h1_{t_i}^{b1}$ lié lorsque ce dernier sera reçu par la réception du signal de synchronisation consécutif audit signal de synchronisation émis ayant provoqué la détermination dudit deuxième horodatage. On comprend de ce qui a été décrit ci-dessus que les émissions des signaux de synchronisation permettent de mettre en œuvre l'étape de transmission E1-1, l'étape de détermination E1-2 des deuxièmes horodatages, et la détermination des premiers horodatages décrites ci-avant.

Par « signal de synchronisation consécutif à un autre », on entend le signal de synchronisation qui arrive directement après sans interposition d'un signal de synchronisation intermédiaire. Par ailleurs, par « signal de synchronisation précédent d'un autre signal de synchronisation », on entend le signal de synchronisation qui arrive directement avant sans interposition d'un signal de synchronisation intermédiaire.

En combinant ladite solution proposée avec la réalisation utilisant la ou les consignes d'asservissement C3, au cours de l'étape de synchronisation E1, chaque signal de synchronisation reçu par le module de radiocommunication 203 du deuxième dispositif 200, et pour lequel le deuxième dispositif 200 dispose d'un des deuxièmes horodatages $h2_{t_i}^{b2}$ déterminé par le compteur de temps 202 du deuxième dispositif 200 lors de la réception d'un signal de synchronisation précédent audit signal de synchronisation SYNC reçu, provoque la mise en œuvre de l'une des étapes de détermination E1-3-1 d'une consigne d'asservissement C3 pour laquelle le différentiel temporel $\delta t$ est obtenu en prenant en compte un des premiers horodatages h1 extrait dudit signal de synchronisation SYNC reçu et ledit deuxième horodatage h2 déterminé par le compteur de temps 202 du deuxième dispositif 200 lors de la réception dudit signal de synchronisation précédent audit signal de synchronisation SYNC reçu. Autrement dit, à la réception du signal de synchronisation SYNC, le premier horodatage qu'il comporte est extrait en vue d'obtenir le différentiel temporel. Ceci présente l'avantage de permettre une comparaison de deux horodatages correspondant respectivement à l'émission et à la réception d'un même signal de synchronisation.

De préférence, l'étape d'asservissement E1-3 peut comporter (figure 3) des étapes E1-3-1 de détermination d'une consigne d'asservissement C3 de l'oscillateur 201 du deuxième dispositif 200 chacune suivie d'une étape d'application E1-3-2 de ladite consigne d'asservissement C3 à l'oscillateur 201 du deuxième dispositif 200. Chaque consigne d'asservissement est alors déterminée en utilisant le différentiel temporel prenant en compte un des premiers horodatages h1, aussi noté $h1_{t_i}^{b1}$, et un des deuxièmes horodatages h2, aussi noté $h2_{t_i}^{b2}$, correspondant respectivement à un horodatage de l'émission et à un horodatage de la réception d'un même signal de synchronisation SYNC. On obtient des valeurs d'horodatages comparables à utiliser pour tendre à l'ajustement du fonctionnement du compteur de temps 202 du deuxième dispositif 200 à celui du premier dispositif 100 d'où il résultera à terme la synchronisation des oscillateurs 101, 201.

De préférence, chaque étape E1-3-1 de détermination d'une consigne d'asservissement de l'oscillateur 201 du deuxième dispositif 200 est déclenchée par la réception d'un des signaux de synchronisation SYNC. Ceci permet d'ajuster de manière plus fine le fonctionnement du compteur de temps du deuxième dispositif 200 à celui du premier dispositif 100. Par ailleurs, ici les signaux de synchronisation SYNC sont émis à intervalles réguliers de sorte à permettre l'application de la consigne d'asservissement C3 déterminée entre la réception du signal de synchronisation ayant déclenché la détermination de ladite consigne d'asservissement C3 et la réception d'un signal de synchronisation consécutif à celui ayant déclenché la détermination de ladite consigne d'asservissement C3. C'est-à-dire que chaque consigne d'asservissement C3 est appliquée avant la réception par le module de radiocommunication 203 d'un signal de synchronisation consécutif au signal ayant déclenché la détermination de ladite consigne d'asservissement C3. Ceci permet que la consigne d'asservissement C3 soit appliquée avant de déterminer un nouveau deuxième horodatage pour prendre en compte l'application de la consigne d'asservissement dans la détermination d'une nouvelle consigne d'asservissement.

On comprend de ce qui a été décrit précédemment que, pour établir le différentiel temporel, le deuxième dispositif 200 doit au préalable disposer du deuxième horodatage correspondant au premier horodatage extrait du signal de synchronisation reçu de sorte que lesdits premier et deuxième horodatages correspondent à ceux déterminés à l'émission et à la réception d'un même signal de synchronisation précédent audit signal de synchronisation reçu. En ce sens, on comprend que le tout premier signal de synchronisation émis ne comportera pas de donnée utile, mais sera envoyé pour permettre la génération du tout premier des premiers horodatages et du tout premier des deuxièmes horodatages. Ensuite, la synchronisation sera effective lorsque le différentiel temporel évoqué précédemment reste dans un intervalle prédéterminé, par exemple entre -100ns et +100ns. La figure 4 permet d'illustrer schématiquement le fonctionnement de l'émission et de la réception des signaux de synchronisation. En figure 4, il est représenté deux lignes verticales, à gauche de la ligne verticale de gauche il est représenté ce qu'il se passe du côté du premier dispositif 100, à droite de la ligne verticale de droite il est représenté ce qu'il se passe du côté du deuxième dispositif 200, et entre les deux lignes verticales il est représenté les signaux de synchronisation. Sur la figure 4, l'étape de synchronisation comporte, pour i = 1, l'émission d'un premier signal de synchronisation noté $SYNC_i(init)$ et permettant l'initialisation dans le sens où son émission par le module de radiocommunication 103 du premier dispositif 100 permet de déterminer un premier horodatage $h1_{t_i}^{b1}$ initial, et où sa réception par le module de radiocommunication 203 du deuxième dispositif 200 permet de déterminer un deuxième horodatage initial $h2_{t_i}^{b2}$. Ensuite, en prenant i = 2 et j = i ⎯ 1, il réalisé un cycle comportant une émission d'un deuxième signal de synchronisation $SYNC_i(h1_{t_j}^{b1})$ contenant la valeur $h1_{t_j}^{b1}$, et dont l'émission par le module de radiocommunication 103 du premier dispositif 100 permet de déterminer un autre pre-

mier horodatage $h1_{t_i}^{b1}$, et dont la réception par le module de radiocommunication 203 du deuxième dispositif 200 permet de déterminer un autre deuxième horodatage $h2_{t_i}^{b2}$. A la réception de $SYNC_i(h1_{t_j}^{b1})$, le cycle est tel que le deuxième dispositif 200 peut extraire la valeur de $h1_{t_j}^{b1}$ du signal de synchronisation reçu, et peut déterminer le différentiel temporel noté $\delta t$ en prenant en compte $h1_{t_j}^{b1}$ et $h2_{t_j}^{b2}$ alors à sa disposition. Ce différentiel temporel $\delta t$ peut alors être utilisé pour déterminer la consigne d'asservissement C3 et l'appliquer à l'oscillateur 201 du deuxième dispositif 200 (figure 2) avant qu'un nouveau signal de synchronisation soit reçu. De manière similaire la synchronisation peut être continuée en répétant le cycle évoqué précédemment notamment avec *i* pouvant aller jusqu'à *n, n* étant un entier positif dépendant du nombre d'itérations du cycle. La valeur de n dépend de la loi de commande qui permet de combiner une bonne précision pour la synchronisation et un transitoire (délai pour mettre en œuvre la synchronisation) pas trop long pour ne pas pénaliser l'application de localisation magnétique. Ainsi, n peut être borné par le besoin applicatif : quelques secondes de transitoire au maximum, soit n pouvant être égal à, ou être de l'ordre de, 1000 en considérant 200 itérations du cycle par seconde.

Selon une mise en œuvre particulière, il est utilisé des signaux de bas-niveau générés par des couches physiques des modules de radiocommunication 103, 203 dans le cadre de la détermination des premiers et deuxièmes horodatages $h1_{t_i}^{b1}$, $h2_{t_i}^{b2}$. La couche physique est la première couche du modèle OSI (de l'anglais « Open Systems Interconnection » ou interconnexion de systèmes ouverts en français). L'avantage de l'utilisation de ces signaux de bas-niveau est qu'ils sont les plus proches des signaux physiques : il y a donc peu ou pas de décalage entre le phénomène physique d'émission, ou de réception, d'un signal, et le transit du signal de bas-niveau, ceci permettant d'assurer la stabilité recherchée de la synchronisation des oscillateurs. En ce sens, les modules de radiocommunication 103, 203, peuvent comporter chacun une couche physique, et :

- pour chaque signal de synchronisation SYNC émis, le module de radiocommunication 103 du premier dispositif 100 génère un signal de bas-niveau IRQ_Tx (figure 1) lorsque sa couche physique réalise l'émission du signal de synchronisation SYNC, cette génération du signal IRQ_Tx provoque la détermination d'un des premiers horodatages $h1_{t_i}^{b1}$ par le compteur de temps 102 du premier dispositif 100,
- pour chaque signal de synchronisation SYNC reçu par le module de radiocommunication 203 du deuxième dispositif 200, il est généré un signal de bas-niveau IRQ_Rx (figure 2) par la couche physique dudit module de radiocommunication 203 du deuxième dispositif 200, cette génération du signal IRQ_Rx provoque la détermination d'un des deuxièmes horodatages $h2_{t_i}^{b2}$ par le compteur de temps 202 du deuxième dispositif 200.

Bien entendu, on comprend de tout ce qui a été décrit précédemment que, préférentiellement, dès qu'un des premiers horodatages est déterminé, ce dernier est transmis au module de radiocommunication 103 du premier dispositif 100 afin d'être envoyé par la suite au deuxième dispositif 200 via un signal de synchronisation correspondant.

L'étape d'asservissement E1-3 de l'oscillateur 201 du deuxième dispositif 200 peut comporter, comme par exemple illustré au mode de réalisation de la figure 2, une utilisation d'un filtre de boucle 207. Le rôle du filtre de boucle 207 est d'élaborer chaque consigne d'asservissement C3 du deuxième oscillateur 201 dont le but est de réduire le différentiel temporel de datation entre les compteurs de temps, ou de maintenir sa valeur absolue en dessous d'un seuil prédéterminé en vue d'assurer la synchronisation recherchée. Ainsi, le filtre de boucle 107 prend en entrée le différentiel temporel, et fournit en sortie une consigne d'asservissement C3 de l'oscillateur 201 du deuxième dispositif 200. Ce seuil prédéterminé est fonction de l'application, par exemple pour une fréquence de signaux sinusoïdaux émis de l'ordre de 20kHz, et un déphasage toléré de plus ou moins 1 degré, le seuil prédéterminé peut être égal à 140ns. En ce sens, on dit que le filtre de boucle 207 rétroagit sur la fréquence de l'oscillateur 201 du deuxième dispositif 200. Par exemple, lorsque la valeur absolue du différentiel temporel est en dessous du seuil prédéterminé, on considère que la synchronisation des oscillateurs 101, 201 est effective.

Chaque consigne d'asservissement C3 peut être une tension de contrôle de l'oscillateur 201 du deuxième dispositif 200. L'oscillateur 201 du deuxième dispositif 200 peut alors être un oscillateur commandé en tension ou « VCO » (pour l'anglais « Voltage Controlled Oscillator »). L'utilisation d'un oscillateur commandé en tension présente l'avantage d'obtenir une granularité très fine sur la fréquence de l'oscillateur asservi, et donc une stabilité in fine très bonne de la fréquence du récepteur 204, le cas échéant de l'émetteur 104, cadencé par l'oscillateur asservi. Préférentiellement, c'est l'oscillateur 201 du deuxième dispositif 200 muni du récepteur 204 qui est asservi car cela permet par exemple, si le système de localisation magnétique comporte plusieurs deuxièmes dispositifs 201 chacun associé à un récepteur 204 correspondant, de synchroniser aisément les oscillateurs reliés aux récepteurs 204 à un même oscillateur relié à l'émetteur 104.

De préférence, le filtre de boucle 207 implémente une loi de commande proportionnelle-intégrale, aussi noté PI, qui permet d'assurer la régulation de l'oscillateur 201 du deuxième dispositif 200 via les consignes d'asservissement C3 successivement déterminées et appliquées. Une telle loi de commande présente l'avantage d'asservir la phase et la fréquence de l'oscillateur asservi, facilitant ainsi la synchronisation en phase et en fréquence des oscillateurs 101, 201 des premier et deuxième dispositifs 100, 200 pour permettre un contrôle ultérieur de la phase des champs magnétiques alternatifs émis et reçus. En effet, une fois la synchronisation obtenue, à la fois la fréquence et la phase du récepteur 204 peuvent être alignées sur celles de l'émetteur 104.

Il a été évoqué précédemment un différentiel temporel $\delta t$ prenant en compte, ou obtenu en prenant en compte, un des premiers horodatages $h1_{t_i}^{b1}$ et un des deuxièmes horodatages $h2_{t_i}^{b2}$. Ce différentiel temporel $\delta t$ peut être déterminé par une étape de détermination du différentiel temporel au cours de l'étape de synchronisation E1. Ce différentiel temporel $\delta t$ peut être un différentiel entre ledit un des premiers horodatages $h1_{t_i}^{b1}$ et ledit un des deuxièmes horodatages $h2_{t_i}^{b2}$, le différentiel peut alors être obtenu par la soustraction dudit un des deuxièmes horodatages $h2_{t_i}^{b2}$ audit un des premiers horodatages $h1_{t_i}^{b1}$, ou inversement. Selon une autre réalisation, on dit que le différentiel temporel $\delta t$ prend en compte aussi un paramètre de décalage (« offset » en anglais). Ainsi, le différentiel temporel $\delta t$ peut être obtenu en soustrayant, audit un des premiers horodatages $h1_{t_i}^{b1}$, ledit un des deuxièmes horodatages $h2_{t_i}^{b2}$ correspondant et le paramètre de décalage. L'avantage de la prise en compte du paramètre de décalage est de tenir compte du fait qu'il existe un délai entre l'instant de génération dudit un des premiers horodatages $h1_{t_i}^{b1}$ et ledit un des deuxièmes horodatages $h2_{t_i}^{b2}$, ce délai étant notamment dû à l'électronique utilisée pour permettre la génération de ces horodatages et au temps de propagation du signal entre les modules de radiocommunication 103, 203 (ce temps de propagation peut par contre être considéré comme négligeable et non pris en compte dans le paramètre de décalage). Dans le cadre de la localisation magnétique, ce paramètre de décalage peut être négligé car il reste généralement constant. Dans le cas où le paramètre de décalage est pris en compte, le générateur de différentiel 206 de la figure 2 peut comporter une entrée supplémentaire (non représentée) fournissant le paramètre de décalage audit générateur de différentiel 206.

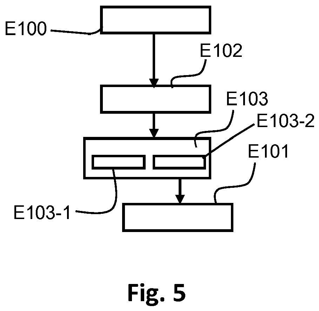

Il résulte de tout ce qui a été décrit précédemment que l'invention peut aussi être relative à un procédé de localisation magnétique, par exemple tel qu'illustré au mode de réalisation selon la figure 5, comportant une étape E100 de mise en œuvre du procédé de synchronisation tel que décrit, et une étape de localisation E101 relative entre les premier et deuxième dispositifs 100, 200 en prenant en compte des données issues du dispositif d'émission et de réception 104, 204 de champs magnétiques alternatifs. Ces données peuvent être par exemple exploitées à la manière de l'enseignement de l'article de RAAB et al. intitulé « Magnetic Position and Orientation Tracking System » publié dans IEEE Transactions on aerospace and electronic systems, Vol. AES-15, No. 5 en septembre 1979 pages 709 à 718. Notamment, pour que la localisation soit la plus juste possible, l'étape de localisation 101 est mise en œuvre alors que les oscillateurs sont synchronisés. Les avantages qui en résultent découlent de ceux décrits ci-avant.

Ainsi, le procédé de localisation magnétique peut comporter, comme illustré en figures 1, 2 et 5, après ajustement du fonctionnement du compteur de temps 202 du deuxième dispositif 200 au fonctionnement du compteur de temps 102 du premier dispositif 100 (c'est-à-dire lorsque les oscillateurs 101, 201 sont synchronisés), une étape de comparaison E102 d'une consigne temporelle C4 avec des données h3, h4 issues des compteurs de temps 102, 202 des premier et deuxième dispositifs 100, 200. Par ailleurs, le procédé de localisation magnétique peut comporter une étape de démarrage E103 du dispositif d'émission et de réception 104, 204 de champs magnétiques alternatifs déclenchée en fonction du résultat de l'étape de comparaison E102. Par exemple, l'étape de démarrage E103 peut être déclenchée dès que la consigne temporelle C4 est atteinte par les données h3 et h4 : la consigne temporelle C4 correspond dans cet exemple à une valeur d'horodatage. Pour mettre en œuvre cela, le premier dispositif 100 peut comporter un comparateur 106 prenant en entrées h3 et C4, et donnant en sortie le cas échéant l'ordre de démarrage O1 transmis sur les entrées En2 des synthétiseurs de fréquence 104a, 104b, 104c de l'émetteur 104. De manière similaire, le deuxième dispositif 200 peut comporter un comparateur 208 prenant en entrées h4 et C4 et donnant en sortie le cas échant l'ordre de démarrage 02 à transmettre sur les entrées En4 des synthétiseurs de fréquence 204a, 204b, 204c du récepteur 204. En ce sens, on comprend que la tenue d'un système de datation en commun (compteurs de temps 102, 202 des premier et deuxième dispositifs 100, 200 ajustés l'un à l'autre) permet le démarrage de signaux alternatifs pour le pilotage de l'émission des champs magnétiques alternatifs et le démarrage des signaux de référence évoqués ci-avant à des instants identiques, ou nonobstant différant d'une tolérance acceptable, d'où il résulte une maîtrise de la phase d'origine des champs magnétiques alternatifs émis et reçus : ainsi, la différence temporelle entre les instants désignés par les deux phases d'origine est minimisée. La consigne temporelle C4 peut être choisie par le premier dispositif 100 et transmise au deuxième dispositif 200 par radiocommunication entre les modules de radiocommunication 103, 203 des premier et deuxième dispositifs 100, 200.

Selon une mise en œuvre du procédé de localisation magnétique (figures 1, 2 et 5), le dispositif d'émission et de réception 104, 204 de champs magnétiques alternatifs comporte l'émetteur 104 de champs magnétiques alternatifs et le récepteur 204 de champs magnétiques alternatifs. Par ailleurs, le procédé de localisation magnétique comporte, après synchronisation des oscillateurs 101, 201 des premier et deuxième dispositifs 100, 200 :

- une étape d'activation E103-1 de l'émetteur 104 de champs magnétiques alternatifs, l'émetteur 104 étant cadencé par l'un des oscillateurs 101, 201 des premier et deuxième dispositifs 100, 200, d'où il résulte l'émission de champs magnétiques alternatifs, et
- une étape d'activation E103-2 du récepteur 204 de champs magnétiques alternatifs, le récepteur 204 étant cadencé par l'autre des oscillateurs 101, 201 des premier et deuxième dispositifs 100, 200, d'où il résulte la réception des champs magnétiques alternatifs émis,

les étapes d'activation des récepteur 204 et émetteur 104 de champs magnétiques alternatifs étant déclenchées (l'étape E103 évoquée précédemment) simultanément en utilisant la consigne temporelle C4 comparée (l'étape E102) évoquée précédemment), d'une part, à la donnée de sortie h3 du compteur de temps 102 du premier dispositif 100, et, d'autre part, à la donnée de sortie h4 du compteur de temps 202 du deuxième dispositif 200. Notamment, les étapes d'activation sont déclenchées lorsque h3 et h4 atteignent la consigne temporelle C4. Ceci permet de maitriser avec une grande précision les références de phase utilisées pour la détection des champs magnétiques alternatifs au niveau du récepteur 204. Ainsi, il est possible d'obtenir une mesure précise de la phase des champs magnétiques reçus (par détection synchrone phase/quadrature par exemple) et de continuer à pouvoir exploiter le cas échéant des méthodes de détection voire de compensation d'artefacts.

Il résulte de ce qui a été décrit ci-avant que l'étape de localisation E101 est notamment mise en œuvre après l'étape de démarrage du dispositif d'émission et de réception 104, 204, ou le cas échéant, après les étapes d'activation des récepteur 204 et émetteur 104.

De préférence, l'étape de synchronisation E1 est déclenchée avant d'activer l'émetteur 104 de champs magnétiques alternatifs et le récepteur 204 de champs magnétiques alternatifs, et est maintenue active au cours du fonctionnement de l'émetteur 104 de champs magnétiques alternatifs et du récepteur 204 de champs magnétiques alternatifs. Ceci présente l'avantage de conserver une synchronisation durant la localisation, et pas seulement au moment de son démarrage pour éviter les phénomènes de glissement de la phase tant que les échanges entre les modules de radiocommunication sont maintenus.

Il résulte de tout ce qui a été dit ci-dessus que l'invention est aussi relative à un système de localisation magnétique en particulier tel que décrit précédemment et adapté au procédé de synchronisation et/ou au procédé de localisation tels que décrits ci-avant. En ce sens, le système de localisation magnétique peut comporter des instructions de code de programme pour l'exécution des étapes du procédé de synchronisation et/ou du procédé de localisation magnétique tels que décrits.

Selon une autre formulation, outre tout ce qui a été dit précédemment en relation avec le système de localisation magnétique, ce dernier peut être configuré de sorte à permettre une synchronisation des oscillateurs 101, 201 des premier et deuxième dispositifs 100, 200 par ajustement, en asservissant l'oscillateur 201 du deuxième dispositif 200, du fonctionnement du compteur de temps 202 du deuxième dispositif 200 au fonctionnement du compteur de temps 102 du premier dispositif 100. Ceci présente l'avantage de permettre la synchronisation évoquée précédemment.

Selon une mise en œuvre particulière du premier dispositif 100, ce dernier est configuré pour émettre des signaux de synchronisation via son module de radiocommunication 103 au module de radiocommunication 203 du deuxième dispositif 200, et pour chaque signal de synchronisation émis, aussi appelé signal de synchronisation courant, le premier dispositif 100 est configuré pour :

- déterminer un premier horodatage lors de l'émission dudit signal de synchronisation,
- transmettre ledit premier horodatage au module de radiocommunication 203 du deuxième dispositif 200 lors de l'émission d'un signal de synchronisation consécutif audit signal de synchronisation courant.

Selon une mise en œuvre particulière du deuxième dispositif 200 (comme par exemple illustrée en figure 2), ce dernier comporte :

- le générateur de différentiel temporel 206 configuré pour prendre en entrées deux horodatages et établir ledit différentiel temporel en prenant en compte lesdits deux horodatages, et, le cas échéant, le paramètre de décalage tel que décrit précédemment,
- le filtre de boucle 207 configuré pour prendre en entrée le différentiel temporel et pour élaborer une consigne d'asservissement C3 pour asservir l'oscillateur 201 du deuxième dispositif 200,
- une mémoire à retard 209 reliée au compteur de temps 202 du deuxième dispositif 200 et au générateur de différentiel temporel 206, ladite mémoire à retard 209 étant configurée pour :

○ recevoir un deuxième horodatage $h2^{b2}_{t_i}$ déterminé par ledit compteur de temps 202 lors de la réception d'un signal de synchronisation par le module de radiocommunication 203 du deuxième dispositif 200,

○ transmettre au générateur de différentiel temporel 206, si la mémoire à retard 209 stocke un deuxième horodatage h2 précédemment reçu, le deuxième horodatage h2 stocké dans ladite mémoire 209 lorsque cette dernière reçoit ledit deuxième horodatage $h2^{b2}_{t_i}$ déterminé.

Par ailleurs, le module de radiocommunication 203 du deuxième dispositif 200 est configuré pour recevoir des signaux de synchronisation et pour :

- extraire d'au moins une partie des signaux synchronisation reçu un premier horodatage h1,
- transmettre ledit premier horodatage h1 extrait en entrée du générateur de différentiel temporel 206.

Il a été décrit l'interaction entre un premier dispositif 100 et un deuxième dispositif 200. Le système de localisation magnétique peut comporter une pluralité de deuxièmes dispositifs 200. L'homme du métier est alors à même d'adapter le procédé de synchronisation du système de localisation magnétique pour permettre de synchroniser tous les oscillateurs 201 des deuxièmes dispositifs 200 à l'oscillateur 101 du premier dispositif 100. Ceci présente l'avantage d'appliquer la présente invention dans le cadre de la localisation de plusieurs deuxièmes dispositifs 200 comportant chacun un récepteur 204 de champs magnétiques alternatifs par rapport au premier dispositif 100 comportant l'émetteur 104 de champs magnétiques alternatifs.

Le canal de radiocommunication utilisé pour envoyer les signaux de synchronisation peut aussi permettre l'envoi de données par exemple de données relatives aux champs magnétiques reçus à destination de la partie du système de localisation magnétique qui comporte l'émetteur de champs magnétiques en vue de mettre en œuvre la localisation relative entre le premier dispositif et le deuxième dispositif. Le canal de communication peut aussi permettre l'envoi de données relatives à des instructions de démarrage du dispositif de localisation magnétique.

Outre les avantages décrits ci-avant, le procédé de synchronisation, le procédé de localisation magnétique, et le système de localisation magnétique décrits permettent :

- par ajustement du fonctionnement du compteur de temps 202 du deuxième dispositif 200 à celui du premier dispositif 100, de conserver la flexibilité d'une solution filaire même avec la présence de deux oscillateurs : ceci permet notamment de conserver au sein du système de localisation un choix quant aux fréquences à utiliser pour générer les champs magnétiques alternatifs par l'émetteur de champs magnétiques alternatifs, notamment intégré au premier dispositif,

- d'exploiter d'éventuelles différences de phase entre des données de réception et d'émission des champs magnétiques alternatifs pour détecter, et éventuellement compenser, des artefacts comme par exemple des perturbateurs de champs magnétiques, l'ambiguïté de phase n'est alors plus limitée à $\pi$ près, et la localisation est donc plus précise,
- une tolérance dans le temps même si le canal de communication entre les modules de radiocommunication est perdu. En effet, la consigne d'asservissement contrôlant l'oscillateur du deuxième dispositif reste alors calée sur la dernière sortie valide de la loi de commande : l'oscillateur reste à cette fréquence lors de l'interruption, et lorsque le canal de communication redevient fonctionnel, le maintien de l'étape de synchronisation lors de la localisation magnétique permet de recaler le fonctionnement du compteur de temps et de l'oscillateur du deuxième dispositif à ceux du premier dispositif,
- d'utiliser un canal de communication entre les premier et deuxième dispositifs déjà présent dans les systèmes usuels de localisation magnétique : il suffit donc d'apporter les modifications nécessaires décrites ci-avant pour mettre en œuvre l'invention.

Tout ce qui a été dit en relation avec le procédé de synchronisation peut s'appliquer au système de localisation, ou au procédé de localisation magnétique, et inversement.

Le procédé de synchronisation, le procédé de localisation et le système de localisation magnétique décrits ci-avant peuvent présenter une application industrielle dans le domaine de la localisation magnétique dans le médical. En particulier, le système de localisation magnétique peut être à six degrés de liberté (en positions et angles) et peut être utilisé dans le domaine médical où l'on cherche par exemple à suivre le déplacement du deuxième dispositif 200 dans un corps par rapport au premier dispositif 100 situé à l'extérieur de ce corps, et notamment fixe par rapport à ce corps. Dans l'exemple d'application au domaine médical, le premier dispositif 100 comportant l'émetteur 104 est fixe à l'extérieur du corps d'un patient et le deuxième dispositif 200 comportant le récepteur 204 peut se déplacer à la surface du corps ou à l'intérieur du corps. Bien entendu, les procédés de synchronisation et de localisation magnétique ainsi que le système de localisation magnétique peuvent être utilisés dans tout domaine mettant en œuvre l'utilisation d'un système de localisation magnétique.

Notamment, lors de l'utilisation du système de localisation magnétique, que cela soit dans le cadre du procédé de synchronisation ou dans le cadre du procédé de localisation magnétique, le premier dispositif 100 reste à une distance du deuxième dispositif 200 (par exemple inférieure au mètre) adaptée à l'intensité du moment magnétique émis par l'émetteur 104 et à la sensibilité du récepteur 204 pour autoriser une localisation magnétique convenable : autrement dit il faut que les champs magnétiques émis puisse être reçus correctement par le récepteur 204. Ceci présente l'avantage de limiter les masquages par des objets tiers, ou le fonctionnement sur de trop longues distances, qui aurait pour conséquence de dégrader les performances de la synchronisation et donc de la localisation magnétique.

On a évoqué ci-dessus un système de localisation magnétique à six dimensions, l'invention peut aussi s'appliquer à tout système de localisation magnétique de dimension moindre (par exemple un système à 5 dimensions, dans le cas où le récepteur ne comporte qu'une seule bobine de réception et dans lequel l'angle selon l'axe de la bobine ne peut être résolu).

## Revendications

1. Procédé de synchronisation d'un système de localisation magnétique, le système de localisation magnétique comportant :

   - un premier dispositif (100) et un deuxième dispositif (200) comportant chacun un oscillateur (101, 201), un compteur de temps (102, 202) cadencé par ledit oscillateur (101, 201), et un module de radiocommunication (103, 203),
   - un dispositif d'émission et de réception (104, 204) de champs magnétiques alternatifs configuré pour permettre une propagation de champs magnétiques alternatifs entre les premier et deuxième dispositifs (100, 200), ledit dispositif d'émission et de réception (104, 204) de champs magnétiques alternatifs étant relié aux oscillateurs (101, 201) des premier et deuxième dispositifs (100, 200),

   **caractérisé en ce qu'**il comporte une étape de synchronisation (E1) configurée pour synchroniser les oscillateurs (101, 201) des premier et deuxième dispositifs (100, 200) par ajustement, en asservissant l'oscillateur (201) du deuxième dispositif (200), du fonctionnement du compteur de temps (202) du deuxième dispositif (200) au fonctionnement du compteur de temps (102) du premier dispositif (100).

2. Procédé de synchronisation selon la revendication 1, **caractérisé en ce que** l'étape de synchronisation (E1) comporte :

   - une étape de transmission (E1-1) de premiers horodatages $(h1_{t_i}^{b1})$, déterminés par le comp-teur de temps (102) du premier dispositif (100), au deuxième dispositif (200) par radiocommunication entre les modules de radiocommunication des premier et deuxième dispositifs (100, 200),
   - une étape de détermination (E1-2) de deuxièmes horodatages $(h2_{t_i}^{b2})$ par le compteur de temps (202) du deuxième dispositif (200),
   - une étape d'asservissement (E1-3) de l'oscillateur (201) du deuxième dispositif (200) utilisant les premiers horodatages $(h1_{t_i}^{b1})$ et les deuxièmes horodatages $(h2_{t_i}^{b2})$.

3. Procédé de synchronisation selon la revendication précédente, **caractérisé en ce que** l'étape de synchronisation (E1) comporte des émissions de signaux de synchronisation (SYNC) par le module de radiocommunication (103) du premier dispositif (100) au module de radiocommunication (203) du deuxième dispositif (200), chacun des premiers horodatages étant transmis par un des signaux de synchronisation (SYNC), et **en ce que** chaque émission de signal de synchronisation (SYNC) par le module de radiocommunication (103) du premier dispositif (100) provoque :

   - une détermination, par le compteur de temps (102) du premier dispositif (100), d'un des premiers horodatages $(h1_{t_i}^{b1})$ à transmettre par un signal de synchronisation consécutif audit signal de synchronisation émis,
   - une détermination, lors d'une réception par le module de radiocommunication (203) du deuxième dispositif (200) dudit signal de synchronisation émis (SYNC), d'un des deuxièmes horodatages $(h2_{t_i}^{b2})$ par le compteur de temps (202) du deuxième dispositif (200).

4. Procédé de synchronisation selon l'une quelconque des revendications 2 à 3, **caractérisé en ce que** l'étape d'asservissement (E1-3) comporte des étapes de détermination (E1-3-1) d'une consigne d'asservissement (C3) de l'oscillateur (201) du deuxième dispositif (200) chacune suivie d'une étape d'application (E1-3-2) de ladite consigne d'asservissement (C3), chaque consigne d'asservissement (C3) étant déterminée en utilisant un différentiel temporel ($\delta t$) prenant en compte un des premiers horodatages (h1) et un des deuxièmes horodatages (h2).

5. Procédé de synchronisation selon la revendication 3, **caractérisé en ce que** l'étape d'asservissement (E1-3) comporte des étapes de détermination

(E1-3-1) d'une consigne d'asservissement (C3) de l'oscillateur (201) du deuxième dispositif (200) chacune suivie d'une étape d'application (E1-3-2) de ladite consigne d'asservissement (C3), chaque consigne d'asservissement (C3) étant déterminée en utilisant un différentiel temporel ($\delta t$) prenant en compte un des premiers horodatages (h1) et un des deuxièmes horodatages (h2), correspondant respectivement à un horodatage de l'émission et à un horodatage de la réception d'un même signal de synchronisation (SYNC).

6. Procédé de synchronisation selon la revendication précédente, **caractérisé en ce que** chaque étape de détermination (E1-3-1) d'une consigne d'asservissement (C3) de l'oscillateur (201) du deuxième dispositif (200) est déclenchée par la réception d'un des signaux de synchronisation (SYNC), et **en ce que** les signaux de synchronisation (SYNC) sont émis à intervalles réguliers de sorte à permettre l'application de la consigne d'asservissement (C3) déterminée entre la réception du signal de synchronisation ayant déclenché sa détermination et la réception d'un signal de synchronisation consécutif.

7. Procédé de synchronisation selon l'une quelconque des revendications 5 à 6 ou les revendications 3 et 4, **caractérisé en ce que** chaque signal de synchronisation (SYNC) reçu par le module de radiocommunication (203) du deuxième dispositif (200), et pour lequel le deuxième dispositif (200) dispose d'un des deuxièmes horodatages déterminé par le compteur de temps (202) du deuxième dispositif (200) lors de la réception d'un signal de synchronisation précédent audit signal de synchronisation reçu, provoque la mise en œuvre de l'une des étapes de détermination (E1-3-1) d'une consigne d'asservissement pour laquelle le différentiel temporel est obtenu en prenant en compte :

    - un des premiers horodatages (h1) extrait dudit signal de synchronisation reçu, et
    - ledit deuxième horodatage (h2) déterminé par le compteur de temps (202) du deuxième dispositif (200) lors de la réception dudit signal de synchronisation précédent audit signal de synchronisation reçu.

8. Procédé de synchronisation selon l'une quelconque des revendications 2 à 7, **caractérisé en ce que** l'étape d'asservissement (E1-3) de l'oscillateur (201) du deuxième dispositif (200) comporte une utilisation d'un filtre de boucle (207).

9. Procédé de synchronisation selon la revendication précédente, **caractérisé en ce que** le filtre de boucle (207) implémente une loi de commande proportionnelle-intégrale.

10. Procédé de synchronisation selon l'une quelconque des revendications précédentes et l'une quelconque des revendications 4 à 5, **caractérisé en ce que** chaque consigne d'asservissement est une tension de contrôle de l'oscillateur (201) du deuxième dispositif (200).

11. Procédé de localisation magnétique, **caractérisé en ce qu'**il comporte une étape (E100) de mise en œuvre du procédé de synchronisation selon l'une quelconque des revendications précédentes, et une étape de localisation (E101) relative entre les premier et deuxième dispositifs (100, 200) en prenant en compte des données issues du dispositif d'émission et de réception (104, 204) de champs magnétiques alternatifs.

12. Procédé de localisation magnétique selon la revendication précédente, **caractérisé en ce qu'**il comporte, après ajustement du fonctionnement du compteur de temps (202) du deuxième dispositif (200) au fonctionnement du compteur de temps (102) du premier dispositif (100), une étape de comparaison (E102) d'une consigne temporelle (C4) avec des données (h3, h4) issues des compteurs de temps (102, 202) des premier et deuxième dispositifs (100, 200), et **en ce qu'**il comporte une étape de démarrage (E103) du dispositif d'émission et de réception (104, 204) de champs magnétiques alternatifs déclenchée en fonction du résultat de l'étape de comparaison (E102).

13. Procédé de localisation magnétique selon l'une quelconque des revendications 11 à 12, **caractérisé en ce que** le dispositif d'émission et de réception (104, 204) de champs magnétiques alternatifs comporte un émetteur (104) de champs magnétiques alternatifs et un récepteur (204) de champs magnétiques alternatifs, et **en ce que** le procédé de localisation magnétique comporte, après synchronisation des oscillateurs (101, 201) des premier et deuxième dispositifs (100, 200) :

    - une étape d'activation (E103-1) de l'émetteur (104) de champs magnétiques alternatifs, cadencé par l'un des oscillateurs (101, 201) des premier et deuxième dispositifs (100, 200), d'où il résulte l'émission de champs magnétiques alternatifs, et
    - une étape d'activation (E103-2) du récepteur (204) de champs magnétiques alternatifs, cadencé par l'autre des oscillateurs (101, 201) des premier et deuxième dispositifs (100, 200), d'où il résulte la réception des champs magnétiques alternatifs émis,

les étapes d'activation des récepteur (204) et émetteur (104) de champs magnétiques alternatifs étant déclenchées simultanément en utilisant une consigne temporelle (C4) comparée, d'une part, à une donnée de sortie (h3) du compteur de temps (102) du premier dispositif (100), et, d'autre part, à une donnée de sortie (h4) du compteur de temps (202) du deuxième dispositif (200).

14. Procédé de localisation magnétique selon la revendication précédente, **caractérisé en ce que** l'étape de synchronisation (E1) est déclenchée avant d'activer les émetteur et récepteur (104, 204), et est maintenue active au cours du fonctionnement des émetteur et récepteur (104, 204).

15. Système de localisation magnétique comportant :

   - un premier dispositif (100) et un deuxième dispositif (200) comportant chacun un oscillateur (101, 201), un compteur de temps (102, 202) cadencé par ledit oscillateur (101, 201), et un module de radiocommunication (103, 203),
   - un dispositif d'émission et de réception (104, 204) de champs magnétiques alternatifs configuré pour permettre une propagation de champs magnétiques alternatifs entre les premier et deuxième dispositifs (100, 200), ledit dispositif d'émission et de réception (104, 204) de champs magnétiques alternatifs étant relié aux oscillateurs (101, 201) des premier et deuxième dispositifs (100, 200),

   **caractérisé en ce qu'**il est configuré de sorte à permettre une synchronisation des oscillateurs (101, 201) des premier et deuxième dispositifs (100, 200) par ajustement, en asservissant l'oscillateur (201) du deuxième dispositif (200), du fonctionnement du compteur de temps (202) du deuxième dispositif (200) au fonctionnement du compteur de temps (102) du premier dispositif (100).

**Patentansprüche**

1. Synchronisationsverfahren eines magnetisches Ortungssystems, wobei das magnetische Ortungssystem Folgendes aufweist:

   - eine erste Vorrichtung (100) und eine zweite Vorrichtung (200), die jeweils einen Oszillator (101, 201), einen Zeitzähler (102, 202), der vom Oszillator (101, 201) getaktet ist, und ein Funkkommunikationsmodul (103, 203) aufweisen,
   - eine Sende- und Empfangsvorrichtung (104, 204) für magnetische Wechselfelder, die dazu ausgebildet ist, eine Ausbreitung magnetischer Wechselfelder zwischen der ersten und zweiten Vorrichtung (100, 200) zu gestatten, wobei die Sende- und Empfangsvorrichtung (104, 204) für magnetische Wechselfelder mit den Oszillatoren (101, 201) der ersten und zweiten Vorrichtung (100, 200) verbunden ist,

   **dadurch gekennzeichnet, dass** es einen Schritt des Synchronisierens (E1) aufweist, der dazu ausgestaltet ist, die Oszillatoren (101, 201) der ersten und zweiten Vorrichtung (100, 200) mittels Anpassung, durch Regelung des Oszillators (201) der zweiten Vorrichtung (200), des Betriebs des Zeitzählers (202) der zweiten Vorrichtung (200) an den Betrieb des Zeitzählers (102) der ersten Vorrichtung (100) zu synchronisieren.

2. Synchronisationsverfahren nach Anspruch 1, **dadurch gekennzeichnet, dass** der Schritt des Synchronisierens (E1) Folgendes aufweist:

   - einen Schritt des Übertragens (E1-1) erster Zeitstempel $\left(h1_{ti}^{b1}\right)$, die vom Zeitzähler (102) der ersten Vorrichtung (100) bestimmt werden, an die zweite Vorrichtung (200) durch Funkkommunikation zwischen den Funkkommunikationsmodulen der ersten und zweiten Vorrichtung (100, 200),
   - einen Schritt des Bestimmens (E1-2) zweiter Zeitstempel $\left(h2_{ti}^{b2}\right)$ durch den Zeitzähler (202) der zweiten Vorrichtung (200),
   - einen Schritt des Regelns (E1-3) des Oszillators (201) der zweiten Vorrichtung (200) mittels der ersten Zeitstempel $\left(h1_{ti}^{b1}\right)$ und der zweiten Zeitstempel $\left(h2_{ti}^{b2}\right)$.

3. Synchronisationsverfahren nach dem vorhergehenden Anspruch, **dadurch gekennzeichnet, dass** der Schritt des Synchronisierens (E1) Sendungen von Synchronisationssignalen (SYNC) durch das Funkkommunikationsmodul (103) der ersten Vorrichtung (100) an das Funkkommunikationsmodul (203) der zweiten Vorrichtung (200) aufweist, wobei jeder der ersten Zeitstempel durch eins der Synchronisationssignale (SYNC) übertragen wird, und dadurch, dass jede Synchronisationssignal-Sendung (SYNC) durch das Funkkommunikationsmodul (103) der ersten Vorrichtung (100) Folgendes bewirkt:

   - ein Bestimmen, durch den Zeitzähler (102) der ersten Vorrichtung (100), eines der ersten Zeitstempel $\left(h1_{ti}^{b1}\right)$, die über ein Synchronisationssignal, das auf das gesendete Synchronisationssignal folgt, zu übertragen ist,
   - ein Bestimmen, bei einem Empfang des gesendeten Synchronisationssignals (SYNC) durch das Funkkommunikationsmodul (203) der zweiten Vorrichtung (200), eines der zweiten Zeitstempel $\left(h2_{ti}^{b2}\right)$ durch den Zeitzähler (202) der zweiten Vorrichtung (200).

4. Synchronisationsverfahren nach einem der Ansprüche 2 bis 3, **dadurch gekennzeichnet, dass** der Schritt des Regelns (E1-3) Schritte des Bestimmens (E1-3-1) einer Regelungsvorgabe (C3) des Oszillators (201) der zweiten Vorrichtung (200) aufweist, auf die jeweils ein Schritt des Anwendens (E1-3-2) der Regelungsvorgabe (C3) folgt, wobei jede Regelungsvorgabe (C3) unter Nutzung eines Zeitunterschieds (δt), unter Berücksichtigung eines der ersten Zeitstempel (h1) und eines der zweiten Zeitstempel (h2) bestimmt wird.

5. Synchronisationsverfahren nach Anspruch 3, **dadurch gekennzeichnet, dass** der Schritt des Regelns (E1-3) Schritte des Bestimmens (E1-3-1) einer Regelungsvorgabe (C3) des Oszillators (201) der zweiten Vorrichtung (200) aufweist, auf die jeweils ein Schritt des Anwendens (E1-3-2) der Regelungsvorgabe (C3) folgt, wobei jede Regelungsvorgabe (C3) unter Nutzung eines Zeitunterschieds (δt) unter Berücksichtigung eines der ersten Zeitstempel (h1) und eines der zweiten Zeitstempel (h2) bestimmt wird, die jeweils einem Zeitstempel der Sendung und einem Zeitstempel des Empfangs eines selben Synchronisationssignals (SYNC) entsprechen.

6. Synchronisationsverfahren nach dem vorhergehenden Anspruch, **dadurch gekennzeichnet, dass** jeder Schritt des Bestimmens (E1-3-1) einer Regelungsvorgabe (C3) des Oszillators (201) der zweiten Vorrichtung (200) durch den Empfang eines der Synchronisationssignale (SYNC) ausgelöst wird, und dadurch, dass die Synchronisationssignale (SYNC) in regelmäßigen Abständen gesendet werden, um die Anwendung der bestimmten Regelungsvorgabe (C3) zwischen dem Empfang des Synchronisationssignals, das deren Bestimmung ausgelöst hat, und dem Empfang eines folgenden Synchronisationssignals zu gestatten.

7. Synchronisationsverfahren nach einem der Ansprüche 5 bis 6 oder 3 bis 4, **dadurch gekennzeichnet, dass** jedes Synchronisationssignal (SYNC), das vom Funkkommunikationsmodul (203) der zweiten Vorrichtung (200) empfangen wird, und für welches die zweite Vorrichtung (200) über einen der zweiten Zeitstempel verfügt, der durch den Zeitzähler (202) der zweiten Vorrichtung (200) beim Empfang eines Synchronisationssignals, das dem empfangenen Synchronisationssignal vorangeht, bestimmt wird, die Durchführung eines der Schritte des Bestimmens (E1-3-1) einer Regelungsvorgabe bewirkt, für die der Zeitunterschied erhalten wird, unter Berücksichtigung:

- eines der ersten Zeitstempel (h1), der aus dem empfangenen Synchronisationssignal extrahiert wird, und
- des zweiten Zeitstempels (h2), der durch den Zeitzähler (202) der zweiten Vorrichtung (200) beim Empfang des Synchronisationssignals, das dem empfangenen Synchronisationssignal vorangeht, bestimmt wird.

8. Synchronisationsverfahren nach einem der Ansprüche 2 bis 7, **dadurch gekennzeichnet, dass** der Schritt des Regelns (E1-3) des Oszillators (201) der zweiten Vorrichtung (200) eine Nutzung eines Schleifenfilters (207) aufweist.

9. Synchronisationsverfahren nach dem vorhergehenden Anspruch, **dadurch gekennzeichnet, dass** der Schleifenfilter (207) ein Proportional-Integral-Regelungsgesetz implementiert.

10. Synchronisationsverfahren nach einem der vorhergehenden Ansprüche und einem der Ansprüche 4 bis 5, **dadurch gekennzeichnet, dass** jede Regelungsvorgabe eine Steuerspannung des Oszillators (201) der zweiten Vorrichtung (200) ist.

11. Magnetisches Ortungsverfahren, **dadurch gekennzeichnet, dass** es einen Schritt (E100) des Durchführens des Synchronisationsverfahrens nach einem der vorhergehenden Ansprüche und einen Schritt des relativen Ortens (E101) zwischen der ersten und zweiten Vorrichtung (100, 200) unter Berücksichtigung der Daten aus der Sende- und Empfangsvorrichtung (104, 204) für magnetische Wechselfelder aufweist.

12. Magnetisches Ortungsverfahren nach dem vorhergehenden Anspruch, **dadurch gekennzeichnet, dass** es nach Anpassung des Betriebs des Zeitzählers (202) der zweiten Vorrichtung (200) an den Betrieb des Zeitzählers (102) der ersten Vorrichtung (100) einen Schritt des Vergleichens (E102) einer Zeitvorgabe (C4) mit Daten (h3, h4) aus den Zeitzählern (102, 202) der ersten und zweiten Vorrichtung (100, 200) aufweist, und dadurch, dass es einen Schritt des Startens (E103) der Sende- und Empfangsvorrichtung (104, 204) für magnetische Wechselfelder aufweist, der je nach dem Ergebnis des Schritts des Vergleichens (E102) ausgelöst wird.

13. Magnetisches Ortungsverfahren nach einem der Ansprüche 11 bis 12, **dadurch gekennzeichnet, dass** die Sende- und Empfangsvorrichtung (104, 204) für magnetische Wechselfelder einen Sender (104) für magnetische Wechselfelder und einen Empfänger (204) für magnetische Wechselfelder aufweist, und dadurch, dass das magnetische Ortungsverfahren nach Synchronisation der Oszillatoren (101, 201) der ersten und zweiten Vorrichtung (100, 200) Folgendes aufweist:

- einen Schritt des Aktivierens (E103-1) des Senders (104) für magnetische Wechselfelder, der von einem der Oszillatoren (101, 201) der ersten und zweiten Vorrichtung (100, 200) getaktet ist, woraus die Sendung magnetischer Wechselfelder resultiert, und
- einen Schritt des Aktivierens (E103-2) des Empfängers (204) für magnetische Wechselfelder, der vom anderen der Oszillatoren (101, 201) der ersten und zweiten Vorrichtung (100, 200) getaktet ist, woraus der Empfang der gesendeten magnetischen Wechselfelder resultiert,

wobei die Schritte des Aktivierens des Empfängers (204) und Senders (104) für magnetische Wechselfelder gleichzeitig unter Nutzung einer Zeitvorgabe (C4) ausgelöst werden, die einerseits mit einem Ausgangsdatenwert (h3) des Zeitzählers (102) der ersten Vorrichtung (100) und andererseits mit einem Ausgangsdatenwert (h4) des Zeitzählers (202) der zweiten Vorrichtung (200) verglichen wird.

14. Magnetisches Ortungsverfahren nach dem vorhergehenden Anspruch, **dadurch gekennzeichnet, dass** der Schritt des Synchronisierens (E1) ausgelöst wird, bevor der Sender und Empfänger (104, 204) aktiviert werden, und im Verlauf des Betriebs des Senders und Empfängers (104, 204) aktiv gehalten wird.

15. Magnetisches Ortungssystem, aufweisend:

- eine erste Vorrichtung (100) und eine zweite Vorrichtung (200), die jeweils einen Oszillator (101, 201), einen Zeitzähler (102, 202), der vom Oszillator (101, 201) getaktet ist, und ein Funkkommunikationsmodul (103, 203) aufweisen,
- eine Sende- und Empfangsvorrichtung (104, 204) für magnetische Wechselfelder, die dazu ausgebildet ist, eine Ausbreitung magnetischer Wechselfelder zwischen der ersten und zweiten Vorrichtung (100, 200) zu gestatten, wobei die Sende- und Empfangsvorrichtung (104, 204) für magnetische Wechselfelder mit den Oszillatoren (101, 201) der ersten und zweiten Vorrichtung (100, 200) verbunden ist,

**dadurch gekennzeichnet, dass** es so ausgebildet ist, dass es eine Synchronisation der Oszillatoren (101, 201) der ersten und zweiten Vorrichtung (100, 200) mittels Anpassung, durch Regelung des Oszillators (201) der zweiten Vorrichtung (200), des Betriebs des Zeitzählers (202) der zweiten Vorrichtung (200) an den Betrieb des Zeitzählers (102) der ersten Vorrichtung (100) gestattet.

**Claims**

1. Method for synchronizing a magnetic locating system, the magnetic locating system comprising:

- a first device (100) and a second device (200) each comprising an oscillator (101, 201), a time counter (102, 202) clocked by said oscillator (101, 201), and a radiocommunication module (103, 203),
- a device (104, 204) for emitting and receiving alternating magnetic fields, said device being configured to allow a propagation of alternating magnetic fields between the first and second devices (100, 200), said device (104, 204) for emitting and receiving alternating magnetic fields being connected to the oscillators (101, 201) of the first and second devices (100, 200),

**characterized in that** it comprises a synchronizing step (E1) configured to synchronize the oscillators (101, 201) of the first and second devices (100, 200) by adjusting, by servo-controlling the oscillator (201) of the second device (200), the operation of the time counter (202) of the second device (200) to the operation of the time counter (102) of the first device (100).

2. Synchronizing method according to Claim 1, **characterized in that** the synchronizing step (E1) comprises:

- a step (E1-1) of transmitting first timestamps $\left(h1_{t_i}^{b1}\right)$, determined by the time counter (102) of the first device (100), to the second device (200) by radiocommunication between the radiocommunication modules of the first and second devices (100, 200),
- a step (E1-2) of determining second timestamps $\left(h2_{t_i}^{b2}\right)$ by the time counter (202) of the second device (200),
- a step (E1-3) of servo-controlling the oscillator (201) of the second device (200) using the first timestamps $\left(h1_{t_i}^{b1}\right)$ and the second timestamps $\left(h2_{t_i}^{b2}\right)$.

3. Synchronizing method according to the preceding claim, **characterized in that** the synchronizing step (E1) comprises the radiocommunication module (103) of the first device (100) emitting synchronization signals (SYNC) to the radiocommunication module (203) of the second device (200), each of the first timestamps being transmitted by one of the synchronization signals (SYNC), and **in that** each time a synchronization signal (SYNC) is emitted by the radiocommunication module (103) of the first device (100):

- one of the first timestamps $\left(h1_{t_i}^{b1}\right)$ to be transmitted by a synchronization signal consecutive to said emitted synchronization signal is determined by the time counter (102) of the first device (100),

- one of the second timestamps $\left(h2_{t_i}^{b2}\right)$ is determined by the time counter (202) of the second device (200), on reception, by the radiocommunication module (203) of the second device (200), of said emitted synchronization signal (SYNC).

4. Synchronizing method according to any one of Claims 2 to 3, **characterized in that** the servo-controlling step (E1-3) comprises steps (E1-3-1) of determining a servo-control setpoint (C3) of the oscillator (201) of the second device (200) each followed by a step (E1-3-2) of applying said servo-control setpoint (C3), each servo-control setpoint (C3) being determined using a time differential ($\delta t$) taking into account one of the first timestamps (h1) and one the second timestamps (h2).

5. Synchronizing method according to Claim 3, **characterized in that** the servo-controlling step (E1-3) comprises steps (E1-3-1) of determining a servo-control setpoint (C3) of the oscillator (201) of the second device (200) each followed by a step (E1-3-2) of applying said servo-control setpoint (C3), each servo-control setpoint (C3) being determined using a time differential ($\delta t$) taking into account one of the first timestamps (h1) and one of the second timestamps (h2), corresponding to a timestamp of the emission and to a timestamp of the reception of the same synchronization signal (SYNC), respectively.

6. Synchronizing method according to the preceding claim, **characterized in that** each step (E1-3-1) of determining a servo-control setpoint (C3) of the oscillator (201) of the second device (200) is triggered by the reception of one of the synchronization signals (SYNC), and **in that** the synchronization signals (SYNC) are emitted at regular intervals so as to allow the application of the determined servo-control setpoint (C3) between the reception of the synchronization signal that triggered its determination and the reception of a consecutive synchronization signal.

7. Synchronizing method according to any one of Claims 5 to 6 or Claims 3 and 4, **characterized in that** each synchronization signal (SYNC) received by the radiocommunication module (203) of the second device (200), and for which the second device (200) has available one of the second timestamps determined by the time counter (202) of the second device (200) on the reception of a synchronization signal preceding said received synchronization signal, causes the implementation of one of the steps (E1-3-1) of determining a servo-control setpoint for which the time differential is obtained while taking into account:

- one of the first timestamps (h1), which is extracted from said received synchronization signal, and
- said second timestamp (h2) determined by the time counter (202) of the second device (200) on the reception of said synchronization signal preceding said received synchronization signal.

8. Synchronizing method according to any one of Claims 2 to 7, **characterized in that** the step (E1-3) of servo-controlling the oscillator (201) of the second device (200) comprises use of a loop filter (207).

9. Synchronizing method according to the preceding claim, **characterized in that** the loop filter (207) implements a proportional-integral control law.

10. Synchronizing method according to any one of the preceding claims and either one of Claims 4 and 5, **characterized in that** each servo-control setpoint is a control voltage of the oscillator (201) of the second device (200).

11. Magnetic locating method, **characterized in that** it comprises a step (E100) of implementing the synchronizing method according to any one of the preceding claims, and a step (E101) of finding the relative location between the first and second devices (100, 200) while taking into account data from the device (104, 204) for emitting and receiving alternating magnetic fields.

12. Magnetic locating method according to the preceding claim, **characterized in that** it comprises, after adjustment of the operation of the time counter (202) of the second device (200) to the operation of the time counter (102) of the first device (100), a step (E102) of comparing a time setpoint (C4) with data (h3, h4) from the time counters (102, 202) of the first and second devices (100, 200), and **in that** it comprises a step (E103) of starting up the device (104, 204) for emitting and receiving alternating magnetic fields, said step being triggered depending on the result of the comparing step (E102).

**13.** Magnetic locating method according to either one of Claims 11 and 12, **characterized in that** the device (104, 204) for emitting and receiving alternating magnetic fields comprises an emitter (104) of alternating magnetic fields and a receiver (204) of alternating magnetic fields, and **in that** the magnetic locating method comprises, after synchronization of the oscillators (101, 201) of the first and second devices (100, 200):

> - a step (E103-1) of activating the emitter (104) of alternating magnetic fields, said emitter being clocked by one of the oscillators (101, 201) of the first and second devices (100, 200), this resulting in the emission of alternating magnetic fields, and
> - a step (E103-2) of activating the receiver (204) of alternating magnetic fields, said receiver being clocked by the other of the oscillators (101, 201) of the first and second devices (100, 200), this resulting in the reception of the emitted alternating magnetic fields,

the steps of activating the receiver (204) and emitter (104) of alternating magnetic fields being triggered simultaneously using a time setpoint (C4) compared, on the one hand, to an output datum (h3) of the time counter (102) of the first device (100), and, on the other hand, to an output datum (h4) of the time counter (202) of the second device (200).

**14.** Magnetic locating method according to the preceding claim, **characterized in that** the synchronizing step (E1) is triggered before the emitter and receiver (104, 204) are activated, and is kept active during the operation of the emitter and receiver (104, 204).

**15.** Magnetic locating system comprising:

> - a first device (100) and a second device (200) each comprising an oscillator (101, 201), a time counter (102, 202) clocked by said oscillator (101, 201), and a radiocommunication module (103, 203),
> - a device (104, 204) for emitting and receiving alternating magnetic fields, said device being configured to allow a propagation of alternating magnetic fields between the first and second devices (100, 200), said device (104, 204) for emitting and receiving alternating magnetic fields being connected to the oscillators (101, 201) of the first and second devices (100, 200),

**characterized in that** it is configured so as to allow a synchronization of the oscillators (101, 201) of the first and second devices (100, 200) by adjustment, by servo-controlling the oscillator (201) of the second device (200), of the operation of the time counter (202) of the second device (200) to the operation of the time counter (102) of the first device (100).

**Fig. 1**

**Fig. 2**

**Fig. 3**

**Fig. 4**

**Fig. 5**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2017131424 A
- US 20050165297 A
- US 8121812 B
- US 20160011013 A

**Littérature non-brevet citée dans la description**

- **RAAB et al.** Magnetic Position and Orientation Tracking System. *IEEE Transactions on aerospace and electronic systems,* Septembre 1979, vol. 15 (5), 709-718